# EUROPEAN PATENT SPECIFICATION

(11) **EP 1 053 302 B1**
(45) Date of publication and mention of the grant of the patent: **28.12.2005**
(21) Application number: 99907460.2
(22) Date of filing: 29.01.1999
(51) Int. Cl.: C12N 5/08

(54) **EXPANDED AND GENETICALLY MODIFIED POPULATIONS OF HUMAN HEMATOPOIETIC STEM CELLS**
VERMEHRTE UND GENETISCH MODIFIZIERTE POPULATIONEN MENSCHLICHER HÄMATOPOITISCHER STAMMZELLEN
POPULATIONS MULTIPLIEES ET GENETIQUEMENT MODIFIEES DE CELLULES SOUCHES HEMATOPOIETIQUES HUMAINES

(30) Priority: 05.02.1998 US 19428; 04.03.1998 US 76836 P; 25.01.1999 US 237291
(43) Date of publication of application: 22.11.2000
(73) Proprietor: Novartis AG, 4056 Basel (CH); Novartis Pharma GmbH, 1230 Wien (AT)
(72) Inventor: MURRAY, Lesley, Jean, San Jose, CA 95129 (US); YOUNG, Judy, Carol, San Carlos, CA 94070 (US); TUSHINSKI, Robert, J., Mountain View, CA 94040 (US)
(74) Representative: Adams, Harvey Vaughan John
(86) International application number: PCT/EP1999/000597
(87) International publication number: WO 1999/040180

(56) References cited:
- EP-A- 0 627 487
- WO-A-97/16535
- MURRAY, L. J. ET AL: "Thrombopoietin combined with FLT3 and KIT ligands increases the transduction efficiency of human primitive hematopoietic progenitors, and novel 'SAR' retroviral vectors allow maintenance of NGFR transgene expression in vivo." BLOOD, (NOV. 15, 1998) VOL. 92, NO. 10 SUPPL. 1 PART 1-2, PP. 466A-467A. MEETING INFO.: 40TH ANNUAL MEETING OF THE AMERICAN SOCIETY OF HEMATOLOGY MIAMI BEACH, FLORIDA, USA DECEMBER 4-8, 1998 THE AMERICAN SOCIETY OF HEAMATOLOGY. , XP002111598
- LAZZARI, L. (1) ET AL: "The combination of TPO, FLT -3, IL - 6 and IL-11 in stroma-free liquid cultures allows extensive expansion and maintenance of hemopoietic stem cells." BLOOD, (NOV. 15, 1998) VOL. 92, NO. 10 SUPPL. 1 PART 1-2, PP. 312B-313B. MEETING INFO.: 40TH ANNUAL MEETING OF THE AMERICAN SOCIETY OF HEMATOLOGY MIAMI BEACH, FLORIDA, USA DECEMBER 4-8, 1998 THE AMERICAN SOCIETY OF HEAMATOLOGY. , XP002111599
- LI K ET AL: "Human neonatal blood: stem cell content, kinetics of CD34+ cell decline and ex vivo expansion capacity." BRITISH JOURNAL OF HAEMATOLOGY, (1999 JAN) 104 (1) 178-85. , XP002111600
- RAMSFJELL, VESLEMOY ET AL: "Thrombopoietin directly and potently stimulates multilineage growth and progenitor cell expansion from primitive (CD34+CD38-) human bone marrow progenitor cells. Distinct and key interactions with the ligands for c-kit and flt3, and inhibitory effects of TGF-.beta. and TNF-.alpha." J. IMMUNOL. (1997), 158(11), 5169-5177 , XP002111601 cited in the application
- YOUNG J C ET AL: "Thrombopoietin stimulates megakaryocytopoiesis, myelopoiesis, and expansion of CD34+ progenitor cells from single CD34+Thy-1+Lin- primitive progenitor cells." BLOOD, (1996 SEP 1) 88 (5) 1619-31. , XP002111602

## Description

### BACKGROUND OF THE INVENTION

Blood cell production derives from a single type of cell, the hematopoietic stem cell, which through proliferation and differentiation gives rise to the entire hematopoietic system. The hematopoietic stem cells are believed to be capable of self-renewal - expanding their own population of stem cells - and they are pluripotent - capable of differentiating into any cell in the hematopoietic system. From this rare cell population, the entire mature hematopoietic system, comprising lymphocytes and myeloid cells (erythrocytes, megakaryocytes, granulocytes and macrophages) is formed. The lymphoid lineage, comprising B cells and T cells, provides for the production of antibodies, regulation of the cellular immune system, detection of foreign agents in the blood, detection of cells foreign to the host, and the like. The myeloid lineage, which includes monocytes, granulocytes, megakaryocytes as well as other cells, monitors for the presence of foreign bodies, provides protection against neoplastic cells, scavenges foreign materials, produces platelets, and the like. The erythroid lineage provides red blood cells, which act as oxygen carriers. Production of mature blood cells is continuous throughout adult life, as the mature cells are short lived. Lifelong production of mature blood cells depends on the activity of the small pool of pluripotent hematopoietic stem cells (HSC) located mainly in the bone marrow.

Pluripotent HSCs are considered to be ideal candidates for disease therapy and ideal target cells for gene therapy. There are many diseases that affect hematopoietic cells for which gene therapy and/or bone marrow transplantation could be useful to alleviate or cure the disease. Such diseases include severe combined immunodeficiency (SCID), chronic myelogenous leukemia (CML), β-thalassemia, sickle cell anemia and the like. Since blood cells have a finite life cycle, gene transfer into more mature hematopoietic cells, such as T cells, at best, provides only transient therapeutic benefit. For example, a SCID patient was treated by introducing a normal ADA gene into her lymphocytes ex vivo and reinjecting the transduced lymphocytes back into the patient (Biotechnology News (1993) 13:14). For effective therapy, ADA-carrying lymphocytes had to be reinjected into the patient every six months. Introducing the ADA gene into HSCs could obviate repeated treatments since the ADA-carrying stem cells could repopulate the bone marrow and completely cure the disease. Thus, gene therapy efforts are focused on HSCs because the transduction and transplantation of these cells could provide a means of ensuring a continuous supply of genetically modified hematopoietic cells during the lifetime of the patient. HSCs are also ultimately responsible for restoring blood cell numbers if the hematopoietic system is depleted in some way.

However, maintaining long-term *ex vivo* cultures of HSCs that remain pluripotent has proven difficult. In addition, for successful gene transduction, the *ex vivo* cultured stem cells must undergo mitosis. (Hajihosseini et al. (1993) *EMBO* **12**:4969; Lewis & Emennan (1994) *J. Virol.* **68**:510). Since the majority of freshly isolated HSC are thought to be quiescent (see, e.g., Ogawa (1993) *Blood* **81**:2844), it is necessary to provide appropriate *ex vivo* conditions to stimulate HSC division without differentiation and subsequent loss of multilineage pluripotential state in order to achieve efficient clinical therapy with gene-manipulated HSC. Stroma appears to be required to provide such conditions, but due to the technical difficulties of using stromal cultures for clinical gene therapy, the appropriate culture conditions which stimulate *ex vivo* replication and maintenance of human HSC in the absence of stroma have not been clearly defined.

To attempt retroviral gene transduction of HSC in the absence of stroma, IL-3 and IL-6 in combination with KL or LIF have been added to stroma-free cultures. (Nolta et al. (1995) *Blood* **86**:101; Junker et al. (1997) *Blood* **89**:4299). The efficiency of gene transduction into these cultured HSCs, however, remains low. In addition, *ex vivo* culture of HSC with IL-3 can be detrimental to maintenance of primitive HSC function, as was indicated by decreased reconstituting ability of HSC in lethally irradiated mice. (see, Knobel et al. (1994) *Exp*. *Hematol*. **22**::1227; Yonemura et al. (1996) *PNAS USA* **93**:4040).

Retrovirus-mediated gene expression in human hematopoietic cells correlated inversely with growth factor stimulation, when cultures included IL-3. In addition, IL-3 can abrogate B lymphoid potential and is a positive regulator of early myelopoiesis. (Hirayama et al. (1994) *PNAS USA* **91**:469). Furthermore, 3-6 days of culture in the presence of IL-3 induces not only cell division of primitive human CD34⁺Lin⁻rhodamine (Rhl23)^{lo} cells, but also differentiation (loss of CD34 expression). (Murray et al. (1995) *Blood* **86**(Suppl. 1):256a:1009; Young et al. (1996) *Blood* **88**:1619). Thus, there is now increasing evidence that inclusion of IL-3 in cultures results in loss of the long term reconstituting ability of HSC. (Knobel et al, *supra;* Yonemura et al (1996) *supra;* Yonemura et al. (1997) *Blood* 89:1915; Dao et al. (1997) *Blood* **89**:446).

Reddy et al. (1995) *Exp. Hematol.* **23**:813, reported that purified bone marrow stem/progenitor cells synchronously progressed from G0/G1 to S phase in vitro in response to a cytokine cocktail consisting of IL-3, IL-1α, bFGF, GM-CSF, G-CSF, CSF-1 and steel factor. Peters et al. (1995) *Exp. Hematol.* **23**:461, described a cytokine cocktail of IL-3, IL-6. IL-11 and SCF used to expand murine hematopoietic progenitor cells in 48 hour *in vitro* cultures of bone marrow. Other cytokine cocktails including thrombopoietin (TPO) have been reported. (Kaushansky et al. (1996) *Exp. Hematol.* **24**:265-9; Ramsfjell et al. (1997) *J*. *Immunol.* **158**:5169-77). However, in some instances, treatment with these and other cocktails of cytokines trigger differentiation of the cell; therefore, pluripotency is lost.

In another approach, mouse bone marrow cells were arrested in G1/G0 phase by culturing the cells in isoleucine-free medium (Reddy et al. (1995) *Exp. Hematol.* **23**:813). Kittler et al. (1994) *Blood* **84**:344A describe a procedure in which isolated murine bone marrow cells are prestimulated in medium containing a cocktail of cytokines for 48 hours, then co-cultured for an additional 48 hours in the same medium and cytokines with the retroviral producer cell line. The cells were then injected into host mice. Kittler's regimen however, produced low levels of engraftment in bone marrow and failed to achieve retroviral transduced cells in the bone marrow or in peripheral blood.

Ramsfjell et al. (1997) *J. Immunol.***158:**5169, reported that thrombopoietin directly stimulates multilineage growth and progenitor cell expansion from primitive human bone marrow progenitor cells. Young et al. (1996) *Blood* **88:** 1619, reported that thrombopoietin stimulates megakaryocytopoiesis, myelopoiesis, and expansion of CD34⁺ progenitor cells from single CD34⁺Thy-1⁺Lin⁻ primitive progenitor cells. WO 97/16535 likewise describes how myeloproliferative leukemia receptor (mpl) ligands, such as thrombopoietin, act on a primitive subpopulation of human stem cells having the characteristics of self-renewal and the ability to give rise to all hematopoietic cell lineages.

For long term efficiency of hematopoietic stem cell therapy, there remains a need for an efficient *ex vivo* non-strornal cell culture system which maintains stem cell pluripotency. It is desirable to achieve an *ex vivo* culture method which results in the induction or activation or HSC cycling without loss of pluripotency. In addition, the method should result in cells suitable for *in vivo* use with minimal toxicity to the individual receiving treatment. The present invention satisfies these needs and provides related advantages as well.

### SUMMARY OF THE INVENTION

The present invention relates to a method for promoting the expansion of hematopoietic stem cells in culture, comprising culturing the cells in the presence of an effective amount of a mpl ligand (such as thrombopoietin (TPO)), a flt3 ligand, and interleukin 6 (IL6). Other cytokines can be added to the culture, alone or in combination, preferably the cytokines are an effective amount of interleukin 3 (IL3), leukemia inhibitory factor (LIF) and/or c-kit ligand. In one such method, the cytokines are TPO, FL. IL6 and LIF. In a further method, the cytokines are TPO, FL, IL6 and IL3. In another method the cytokines are TPO, FL, IL6, LIF and IL3. In yet another method, the cytokines are TPO, FL, IL6, and a c-kit ligand. In still another method, the cytokines are, TPO, FL, IL6, LIF, IL3 and a c-kit ligand. In yet another method, the cytokines are TPO, FL, IL6, LIF and a c-kit ligand.

The methods of culturing described herein give rise to populations of hematopoietic stem cells characterized by the capability of self-renewal and the ability to give rise to all hematopoietic cell lineages. In one such method, the hematopoietic stem cells are human hematopoietic stem cells, preferably CD34⁺, more preferably, CD34⁺Thy-1⁺ and even more preferably, CD34⁺Thy-1⁺Lin⁻.

The present invention also relates to a method for restoring hematopoietic capability in a subject, comprising: (a) culturing a population comprising hematopoietic stem cells in the presence of a mpl ligand, particularly TPO, a flt3 (FL) ligand, and interleukin 6 (IL6) under conditions which favor expansion of the hematopoietic stem cell population; and (b) administering an effective amount of said expanded population of stem cells to the subject. Other cytokines can also be added, alone or in combination, for example an effective amount of leukemia inhibitory factor (LIF), interleukin 3 (IL3) and/or a c-kit ligand. In one such method, the cytokines are TPO, FL, IL6, and LIF. In another method, the cytokines are TPO, FL, IL6 and IL3. In yet another method, the cytokines are TPO, FL, IL6, IL3 and LIF. In a further method, the cytokines are TPO, FL, IL6 and a c-kit ligand. In yet another method, the cytokines are TPO, FL, IL6, UF and a c-kit ligand. Another method comprises the cytokines TPO, FL, IL6, IL3 and a c-kit ligand or TPO, FL, IL6, LIF, IL3 and a c-kit ligand. The hematopoietic stem cells may be human hematopoietic stem cells, preferably CD34⁺, more preferably, CD34⁺Thy-1⁺ and even more preferably, CD34⁺Thy-1⁺Lin⁻. The hematopoietic stem cells cultured in the cytokines and administered to the subject may be allogeneic. The hematopoietic stem cells cultured in the cytokines and administered to the subject may be autologous.

In a first aspect, the invention provides a method for modifying a hematopoietic stem cell, comprising contacting a gene delivery vehicle comprising a polynucleotide sequence with a population of hematopoietic stem cells cultured with fibronectin or RetroNectin™ and in the presence of an effective amount of a mpl ligand and a flt3 ligand (FL). Other molecules can also be added, alone or in combination, to the culture, for example, a c-kit ligand, interleukin 6 (IL6), interleukin 3 (IL3), leukemia inhibitory factor (LIF) and/or fibronectin. In one embodiment, the added molecules are a TPO, FL, and a c-kit ligand. In a second embodiment, the added molecules are TPO, FL and IL6. In a third embodiment, the added molecules are TPO, FL, IL6 and LIF. In a fourth embodiment, the added molecules are TPO, FL, IL6, LIF and IL3. In a fifth embodiment, the added molecules are TPO, FL, IL6, IL3 and a c-kit ligand: In a sixth embodiment, the added molecules are TPO, FL. IL6, EL3, a c-kit ligand and LIF. In a further embodiment, the molecules are TPO, FL, IL6, IL.3. In any of these culture conditions, the polynucleotide sequence encodes a product selected from the group consisting of a peptide, a ribozyme and an antisense sequence and the gene delivery vehicle is selected from the group consisting of a retroviral vector, a DNA vector and a liposomal delivery vehicle. Further embodiments of the present invention are set out in the appendant claims.

### BRIEF DESCRIPTION OF THE DRAWINGS

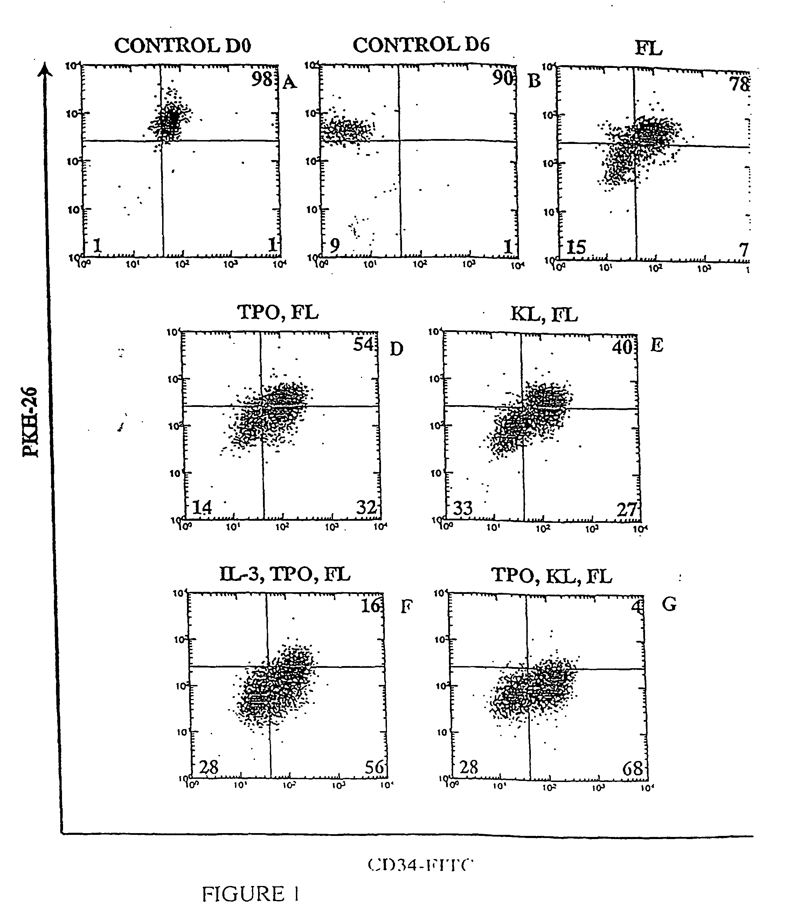

Figure 1, panels A through G, depict FACS analysis of PKH26-stained CD34⁺Thy-I⁺Lin⁻ bone marrow (BM) cells for 6 days in various cytokine combinations containing FL. The numbers show the percentages of cells in each quadrant from a representative experiment. Loss of PKH26 fluorescence indicates cell division. Loss of CD34 expression. indicates differentiation. Control cells were cultured overnight (D0) or for 6 days (D6) without cytokines and quadrants were set using live-gated undivided cells. The percentages of undivided cells (UR and UL quadrants) are combined.

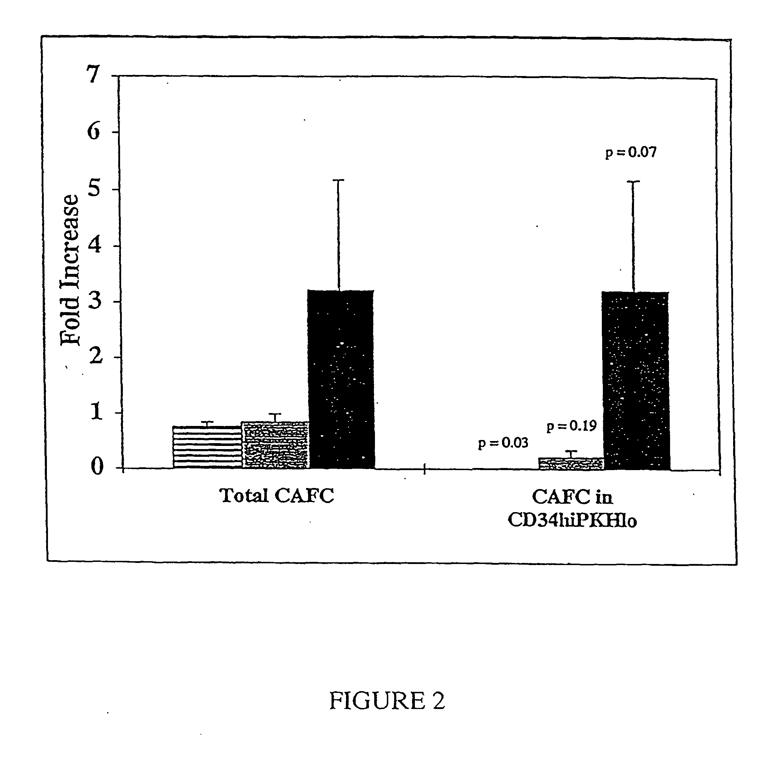

Figure 2 illustrates fold increase in either total CAFC or CAFC within a FACS sorted CD34^{hi}PKH^{lo} cell population from cultures of BM CD34⁺Thy-I⁺Lin⁻ cells with various cytokines. Striped bars depict IL3, 1L6 and LIF treatment. Grey bars depict TPO and KL treatment. Solid bars depict TPO, FL and KL treatment.

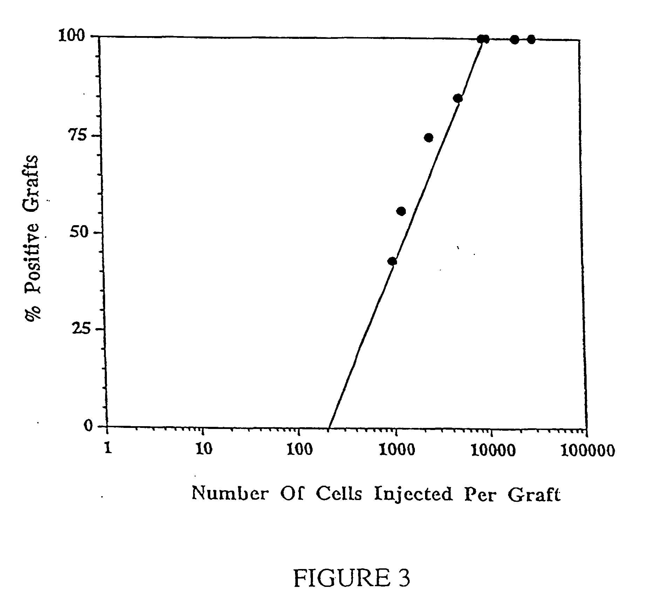

Figure 3 illustrates the percentage of positive grafts versus number of uncultured . CD34⁺Thy-1⁺Lin⁻ (BM) cells injected per grafts. This was done to choose dose of 10K Thy-1⁺ cells.

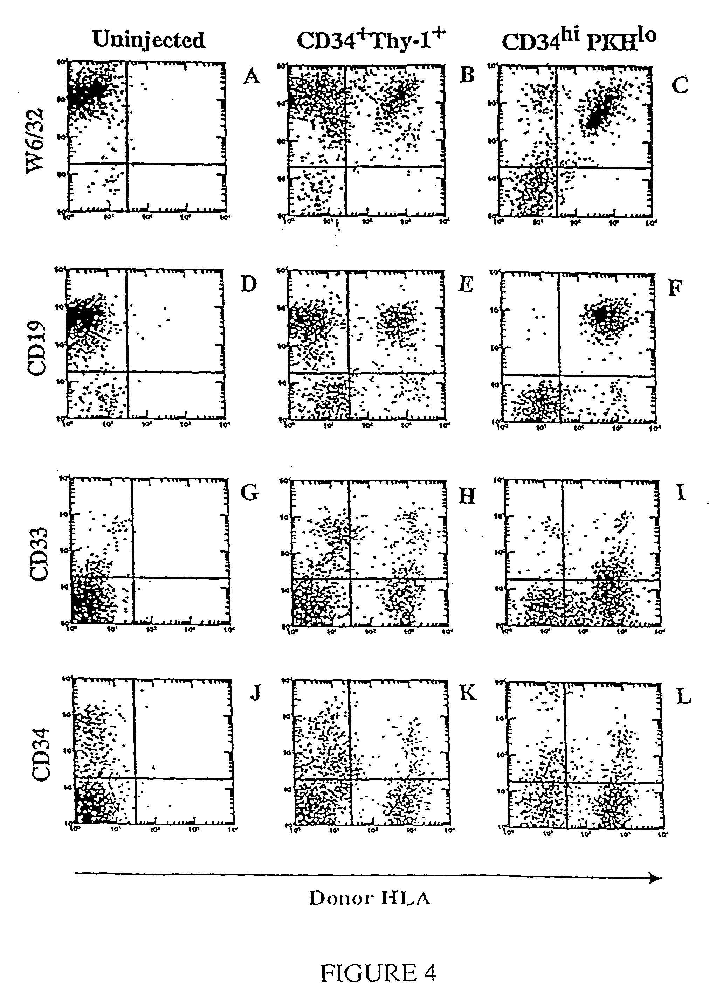

Figure 4, panels A through L, show FACS analysis of donor human cell engraftment 8 weeks after SCID-hu bone injection of 10,000 cells per graft. CD34⁺ cells which divided during 6 days of culture in TPO, KL and FL (CD34⁺ PKH^{lo}) retained their capacity for in vivo marrow repopulation (panel C, F. I, L) when compared to uncultured BM CD34⁺Thy-1⁺Lin (panel B; E, H, K). The x-axis shows staining for donor HLA allotype. The y-axis shows staining for total human cells (W6/32, anti-human class I MHC) or lineage markers.

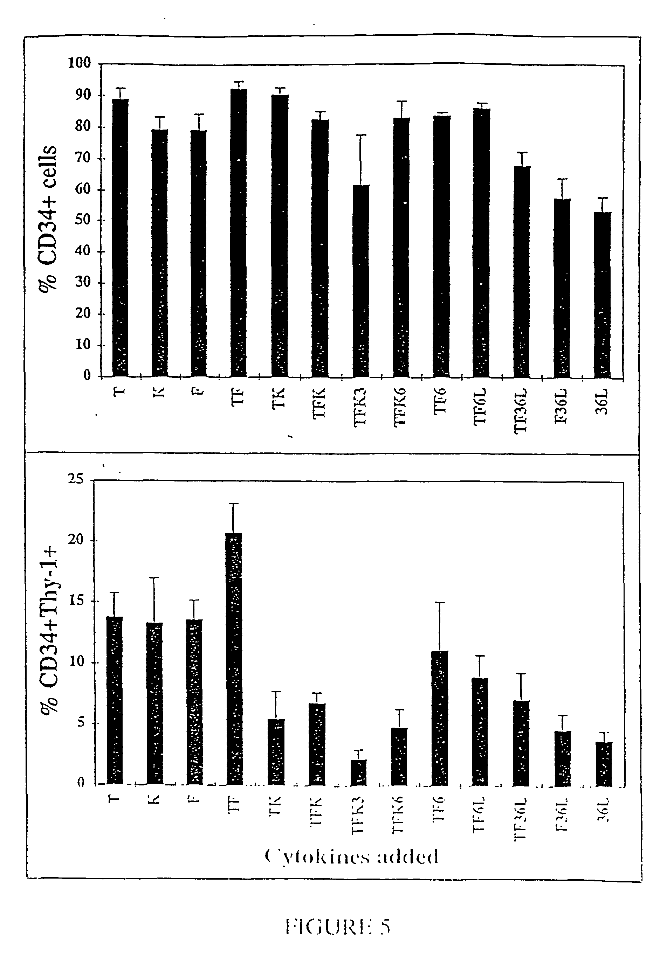

Figure 5 shows the % CD34⁺ and % CD34⁺Thy-1⁺ cells determined from FACS dot plots from MPB CD34⁺ cells cultured for 90 hours. Columns are the means of 3-12 experiments on cells from different normal donors, and error bars are the standard errors of the mean. T = thrombopoietin, K = kit ligand, F = flt3 ligand. L = LIF, 3 = IL-3, 6 = IL-6.

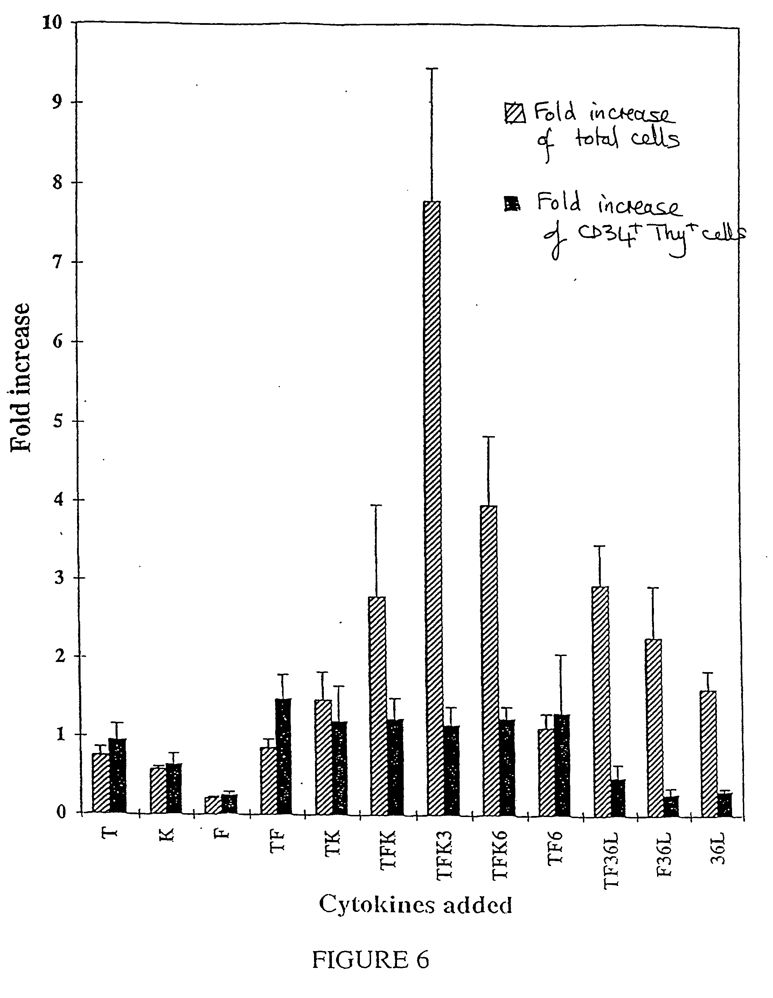

Figure 6 shows the fold increase of CD34⁺Thy-1⁺ cells at 90 hours calculated from the total number of CD34⁺Thy-1⁺ cells at day 0 and at the end of the culture. Columns are the means of 3-12 experiments, and error bars are the standard errors of the mean. Hatched bars represent total cells and solid bars represent CD34⁺⁺Thy-1⁺ cell numbers. T = thrombopoietin, K = kit ligand, F = flt3 ligand, L = LIF, 3 = IL-3, 6 = IL-6.

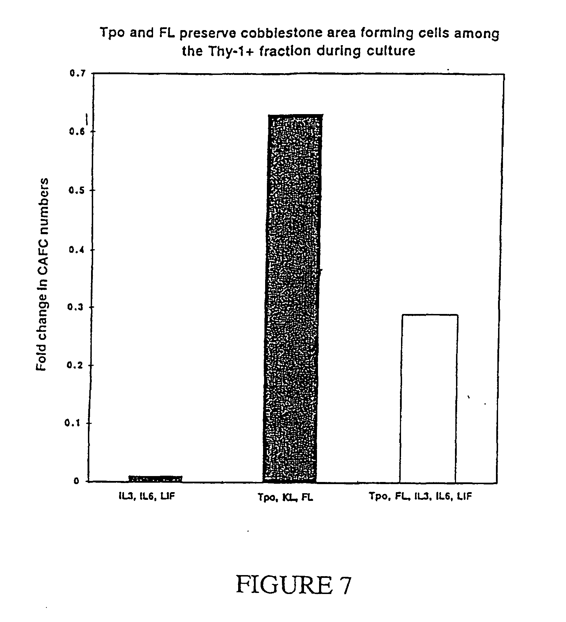

Figure 7 shows the fold change in CAFC numbers among MPB cells cultured for 5 days in various cytokines CD34⁺Thy-1⁺ cells compared to uncultured cells.

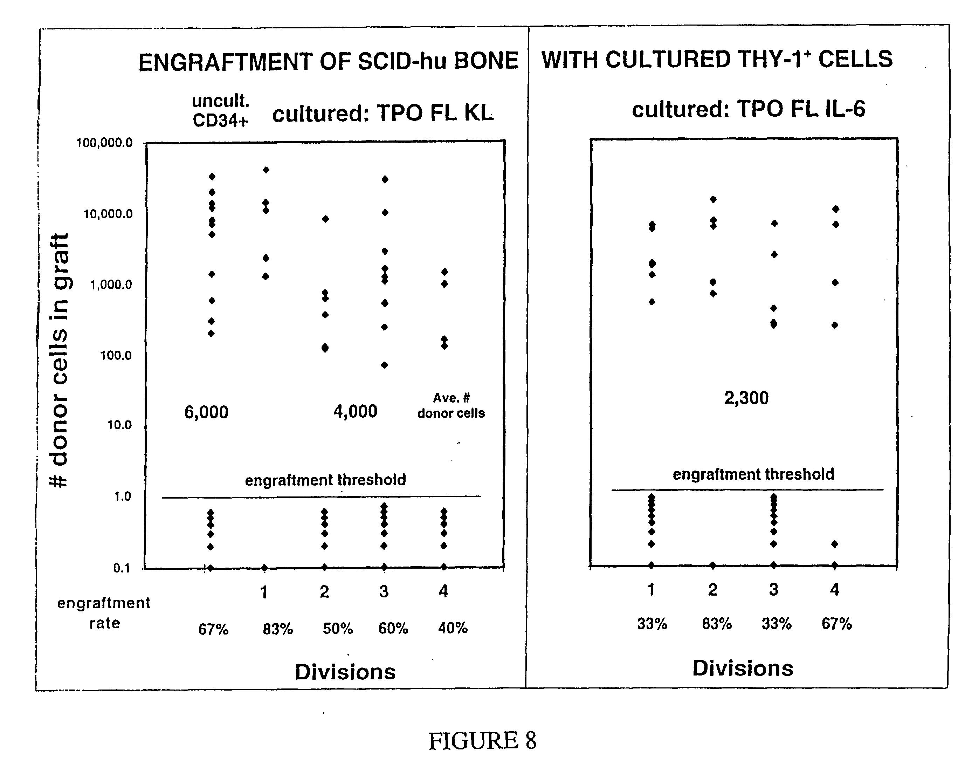

Figure 8 illustrates engraftment of SCID-hu bone with CFSE labeled CD34⁺MPB cells cultured for 5 days. Cultured CD34⁺ cells were sorted, segregating individual divisions by means of CFSE dye retention. Each data point represents an individual bone graft. Grafts containing less than 1% human donor cells out of a total cells in the graft were considered to have no engraftment. Typically at least 10,000 cells (both human and mouse) could be obtained from the grafts, therefore 1% represented at least 100 cellular events.

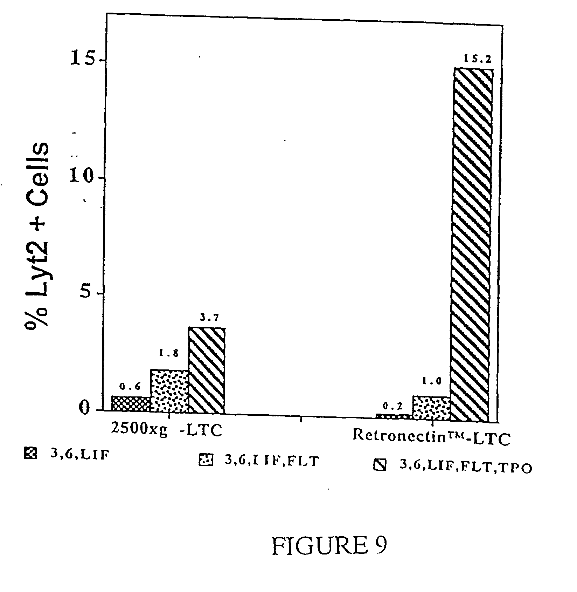

Figure 9 shows Lyt2 transgene expression after long-term *in vitro* stromal culture of transduced MPB CD34⁺ cells.

### MODES FOR CARRYING OUT THE INVENTION

The practice of the present invention will employ, unless otherwise indicated, conventional techniques of molecular biology, microbiology, cell biology and recombinant DNA, which are within the skill of the art. *See, e.g.,* Sambrook, Fritsch, and Maniatis, MOLECULAR CLONING: A LABORATORY MANUAL, 2nd edition (1989); CURRENT PROTOCOLS IN MOLECULAR BIOLOGY, (F.M. Ausubel et al. eds., 1987); the series METHODS IN ENZYMOLOGY (Academic Press, Inc.); PCR 2: A PRACTICAL APPROACH (M.J. McPherson, B.D. Hames and G.R. Taylor eds., 1995); ANIMAL CELL CULTURE (R.I. Freshney. Ed., 1987); and ANTIBODIES: A LABORATORY MANUAL (Harlow et al. eds., 1987).

### Definitions

As used herein, certain terms will have defined meanings.

The term "hematopoietic stem cell" refers to animal, especially mammalian, preferably human, hematopoietic stem cells and not stem cells of other cell types. "Stem cells" also refers to a population of hematopoietic cells having all of the long-term engrafting potential *in vivo.* Animal models for long-term engrafting potential of candidate human hematopoietic stem cell populations include the SCID-hu bone model (Kyoizumi et al. (1992) *Blood* **79**:1704; Murray et al. (1995) *Blood* 85(2) 368-378) and the *in utero* sheep model (Zanjani et al. (1992) *J*. *Clin*. *Invest.* **89**:1179). For a review of animal models of human hematopoiesis, see Srour et al. (1992) *J. Hematother.* **1**:143-153 and the references cited therein. At present, the best *in vitro* assay for stem cells is the long-term culture-initiating cell (LTCIC) assay, based on a limiting dilution analysis of the number of clonogenic cells produced in a stromal co-culture after 5-8 weeks. (Sutherland et al. (1990) *Proc. Nat'l Acad. Sci.* **87**:3584-3588). The LTCIC assay has been shown to correlate with another commonly used stem cell assay, the cobblestone area forming cell (CAFC) assay, and with long-term engrafting potential *in vivo.* (Breems et al. (1994) *Leukemia* **8**:1095). Exemplary of a highly enriched stem cell population is a population having the CD34⁺Thy-1⁺LIN⁻ phenotype as described in U.S. Patent No. 5,061,620. A population of this phenotype will typically have an average CAFC frequency of approximately 1 /20. Murray et al. (1995) *supra;* Lansdorp et al. (1993) *J. Exp. Med.* **177**:1331. It will be appreciated by those of skill in the art that the enrichment provided in any stem cell population will be dependent both on the. selection criteria used as well as the purity achieved by the given selection techniques.

As used herein, the term "expansion" is intended to mean allowance of progenitor cells to increase in cell number from the pluripotent stem cells used to initiate the culture. The term "survival" refers to the ability to continue to remain alive or function.

The hematopoietic stem cells used to inoculate the cell culture may be derived from any source including bone marrow, both adult and fetal, cytokine or chemotherapy mobilized peripheral blood, fetal liver, bone marrow or umbilical cord blood.

In general, it is desirable to isolate the initial inoculation population from neoplastic cells prior to culture. Separation of stem cells from neoplastic cells can be performed by any number of methods, including cell sorters, magnetic beads, packed columns. Isolation of the phenotype (CD34⁺Thy-1⁺CD14⁻CD15⁻) from multiple myeloma patients has been shown to reduce the tumor burden to less than 1 tumor per 10⁵ purified cells.

As used in the specification and claims, the singular form "a", "an" and "the" include plural references unless the context clearly dictates otherwise. For example, the term "a stem cell" includes a plurality of cells, including mixtures thereof, and reference to "a flt3 ligand" or "mpl ligand" include compounds able to bind to the flt3 or mpl receptor with sufficient specificity to elicit flt3- or TPO-mediated biological activity.

As used herein, the term "cytokine" refers to any one of the numerous factors that exert a variety of effects on cells, for example, inducing growth or proliferation. Non-limiting examples of cytokines which may be used alone or in combination in the practice of the present invention include, interleukin-2 (IL-2), stem cell factor (SCF), interleukin 3 (IL-3), interleukin 6 (IL-6) including soluble IL-6 receptor, interleukin 12 (IL12), G-CSF, granulocyte-macrophage colony stimulating factor (GM-CSF), interleukin 1 alpha (IL-1α), interleukin 11 (IL-11), MIP-1α, leukemia inhibitory factor (LIF), c-kit ligand, thrombopoietin (TPO) and flt3 ligand. The present invention also includes culture conditions in which one or more cytokine is specifically excluded from the medium. Cytokines are commercially available from several vendors such as, for example, Amgen (Thousand Oaks, CA), R &D Systems and Immunex (Seattle, WA). It is intended, although not always explicitly stated, that molecules having similar biological activity as wild-type or purified cytokines (e.g., recombinantly produced) are intended to be used.

The terms "mpl (myleoproliferate leukemia) ligand" "flt3 ('FL') ligand" and "c-kit ('KL') ligand" are meant any compounds capable of binding to the mpl, flt3 (FL) and c-kit (KL, also'called steel factor (Stl), mast cell growth factor (MGF)) receptors respectively such that one or more biological actions associated with the binding of the wild-type receptor are initiated. Biological activity includes (1) promotion of the survival of stem cells in culture, such that the cell maintains the capability of self-renewal and the ability to give rise to all hematopoietic cell lineages, (2) expansion of stem cell populations such that the cell maintains the capability of self-renewal and the ability to give rise to all hematopoietic cell lineages and (3) activation of a quiescent stem cell, such that the stem cell is activated to divide and the resulting cells maintain the capability of self-renewal and the ability to give rise to all hematopoietic cell lineages.

The mpl, flt3 and c-kit ligands also include antibodies to these receptors capable of binding to the appropriate receptor such that one or more of the above-described biologically mediated actions are initiated. Such antibodies may be pooled monoclonal antibodies with different epitopic specificities, or be distinct monoclonal antibodies. The terms "mpl ligand", "flt3 ligand" or "c-kit ligand" further include "mimetic" molecules, that is small molecules that are able to bind these receptors such that one or more of the above-described biological actions are initiated. An example of a TPO mimetic is found in Cwirla et al. (1997) *Science* 276:1696.

Other molecules can be added to the culture media, for instance, adhesion molecules, such as fibronectin or RetroNectin™ (commercially produced by Takara Shuzo Co. Ltd., Otsu Shigi, Japan). The term "fibronectin" refers to a glycoprotein that is found throughout the body and its concentration is particularly high in connective tissues where it forms a complex with collagen. Fibronectin is thought to play a role in controlling cell growth and differentiation and in cell adhesion.

The term "culturing" refers to the propagation of cells or organisms on or in media of various kinds. It is understood that the descendants of a cell grown in culture may not be completely identical (either morphologically, genetically, or phenotypically) to the parent cell.

An "effective amount" is an amount sufficient to effect beneficial or desired results. An effective amount can be administered in one or more administrations. For purposes of this invention, an effective amount of the cytokines used herein is an amount that is sufficient to promote survival, expansion and/or transduction (with a gene delivery vehicle) of hematopoietic stem cells. In one embodiment, an effective amount of the various cytokines individually may be from about 0.1 ng/mL to about 500 ng/mL, usually from about 5 ng/mL to about 200 ng/mL, and even more usually from about 10 ng/mL to about 100 ng/mL. In another embodiment, the cytokines are contained in the media and replenished by media perfusion.

An "isolated" or "purified" population of cells is substantially free of cells and materials with which it is associated in nature. By substantially free or substantially purified is meant at least 50% of the population are hematopoietic stem cells, preferably at least 70%, more preferably at least 80%, and even more preferably at least 90% free of non-pluripotent cells with which they are associated in nature. "Substantially free of stromal cells" shall mean a cell population which, when placed in a culture system as described herein, does not form an adherent cell layer.

A "subject" is a vertebrate, preferably a mammal, more preferably a human. Mammals include, but are not limited to, mice, monkeys, farm animals, sport animals, and pets.

As used herein, a "genetic modification" refers to any addition, deletion or disruption to a cell's normal nucleotides. The methods of this invention are intended to encompass any method of gene transfer into hematopoietic stem cells, including but not limited to viral mediated gene transfer, liposome mediated transfer, transformation, transfection and transduction, e.g., viral mediated gene transfer such as the use of vectors based on DNA viruses such as adenovirus, adeno-associated virus and herpes virus, as well as retroviral based vectors. A "viral vector" is defined as a recombinantly produced virus or viral particle that comprises a polynucleotide to be delivered into a host cell, either *in vivo, ex* vivo or *in vitro.* Examples of viral vectors include retroviral vectors such as lentiviral vectors; adenovirus vectors; adeno-associated virus vectors and the like. In aspects where gene transfer is mediated by a retroviral vector, a vector construct refers to the polynucleotide comprising the retroviral genome or part thereof, and a therapeutic gene.

As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, retroviral vector refers to a viral particle capable of introducing exogenous nucleic acid into a cell through a viral or viral-like entry mechanism.

Retroviruses carry their genetic information in the form of RNA; however, once the virus infects a cell, the RNA is reverse-transcribed into the DNA form which integrates into the genomic DNA of the infected cell. The integrated DNA form is called a provirus.

In aspects where gene transfer is mediated by a DNA viral vector, such as a adenovirus (Ad) or adeno-associated virus (AAV), a vector construct refers to the polynucleotide comprising the retroviral genome or part thereof, and a therapeutic gene. Adenoviruses (Ads) are a relatively well characterized, homogenous group of viruses, including over 50 serotypes. (see, *e.g.,* WO 95/27071) Ads are easy to grow and do not require integration into the host cell genome. Recombinant Ad-derived vectors, particularly those that reduce the potential for recombination and generation of wild-type virus, have also been constructed. (see, WO 95/00655; WO 95/11984).

Adeno-associated virus (AAV) has also been used as a gene transfer system. (See, *e.g.,* U.S. Patent Nos. 5,693,531 and 5,691,176). Wild-type AAV has high infectivity and specificity in integrating into the host cells genome. (Hermonat and Muzyczka (1984) *PNAS* USA 81:6466-6470; Lebkowski *et al.* (1988) *Mol. Cell. Biol.* 8:3988-3996). Recombinant AAV vectors have been produced in high titers and which can transduce target cells at high efficiency.

Vectors that contain both a promoter and a cloning site into which a polynucleotide can be operatively linked are well known in the art. Such vectors are capable of transcribing RNA *in vitro* or *in vivo,* and are commercially available from sources such as Stratagene (La Jolla, CA) and Promega Biotech (Madison, WI). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression. Examples of vectors are viruses, such as baculovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression.

Among these are several non-viral vectors, including DNA/liposome complexes, and targeted viral protein DNA complexes. To enhance delivery to a cell, the nucleic acid or proteins of this invention can be conjugated to antibodies or binding fragments thereof which bind cell surface antitens, e.g., TCR, CD3 or CD4. Liposomes that also comprise a targeting antibody or fragment thereof can be used in the methods of this invention. This invention also provides the targeting complexes for use in the methods disclosed herein.

Polynucleotides are inserted into vector genomes using methods well known in the art. For example, insert and vector DNA can be contacted, under suitable conditions, with a restriction enzyme to create complementary ends on each molecule that can pair with each other and be joined together with a ligase. Alternatively, synthetic nucleic acid linkers can be ligated to the termini of restricted polynucleotide. These synthetic linkers contain nucleic acid sequences that correspond to a particular restriction site in the vector DNA. Additionally, an oligonucleotide containing a termination codon and an appropriate restriction site can be ligated for insertion into a vector containing, for example, some or all of the following: a selectable marker gene, such as the neomycin gene for selection of stable or transient transfectants in mammalian cells; enhancer/promoter sequences from the immediate early gene of human CMV for high levels of transcription; transcription termination and RNA processing signals from SV40 for mRNA stability, SV40 polyoma origins of replication and ColE1 for proper episomal replication; versatile multiple cloning sites; and T7 and SP6 RNA promoters for *in vitro* transcription of sense and antisense RNA. Other means are well known and available in the art.

### Ex Vivo Cultures of Hematopoietic Stem Cells

In one embodiment, the method of the present invention comprises the expansion and/or survival of a hematopoietic stem cell (HSC) culture. Cell populations useful in providing a source of HSCs for this method include, and are not limited to, cell populations obtained from bone marrow, both adult and fetal, mobilized peripheral blood (MPB) and umbilical cord blood. Hematopoietic stem cells can be isolated from any known source of hematopoietic stem cells, including, but not limited to, bone marrow, both adult and fetal, mobilized peripheral blood (MPB) and umbilical cord blood. The use of umbilical cord blood is discussed, for instance, in Issaragrishi et al. (1995) *N. Engl. J. Med.* **332:**367-369. Initially, bone marrow cells can be obtained from a source of bone marrow, including but not limited to, ilium (e.g., from the hip bone via the iliac crest), tibia, femora, vertebrate, or other bone cavities. Other sources of stem cells include, but are not limited to, embryonic yolk sac, fetal liver, and fetal spleen.

The methods can include further enrichment or purification procedures or steps for stem cell isolation by positive selection for other stem cell specific markers. Suitable positive stem cell markers include, but are not limited to, CD34⁺ and Thy-1⁺. Preferably the hematopoietic cells are human but can be derived from any suitable animal, e.g., human, simian, porcine or murine.

For isolation of bone marrow, an appropriate solution can be used to flush the bone, including, but not limited to, salt solution, conveniently supplemented with fetal calf serum (FCS) or other naturally occurring factors, in conjunction with an acceptable buffer at low concentration, generally from about 5-25 mM. Convenient buffers include, but are not limited to, HEPES, phosphate buffers and lactate buffers. Otherwise bone marrow can be aspirated from the bone in accordance with conventional techniques.

Preferably, the source of hematopoietic stem cells is initially subject to negative selection techniques to remove those cells that express lineage specific markers and retain those cells which are lineage negative ("Lin⁻"). Lin⁻ cells generally refer to cells which lack markers such as those associated with T cells (such as CD2, 3, 4 and 8), B cells (such as CD10, 19 and 20), myeloid cells (such as CD14, 15, 16 and 33), natural killer ("NK") cells (such as CD2, 16 and 56), RBC (such as glycophorin A), megakaryocytes (CD41), mast cells, eosinophils or basophils. Methods of negative selection are known in the art. The absence or low expression of such lineage specific markers is identified by the lack of binding of antibodies specific to the cell specific markers, useful in so-called "negative selection". Preferably the lineage specific markers include, but are not limited to, at least one of CD2, CD14, CD15, CD16, CD19, CD20, CD38, HLA-DR and CD71; more preferably, at least one of CD14, CD15 and CD 19. As used herein, Lin refers to a cell population selected based on the lack of expression of at least one lineage specific marker. A highly enriched composition can be obtained by selective isolation of cells that are CD34⁺Lin.

Various techniques can be employed to separate the cells by initially removing cells of dedicated lineage. Monoclonal antibodies are particularly useful for identifying markers associated with particular cell lineages and/or stages of differentiation. The antibodies can be attached to a solid support to allow for crude separation. The separation techniques employed should maximize the retention of viability of the fraction to be collected. Various techniques of different efficacy can be employed to obtain "relatively crude" separations. Such separations are up to 10%, usually not more than about 5%, preferably not more than about 1%, of the total cells present not having the marker can remain with the cell population to be retained. The particular technique employed will depend upon efficiency of separation, associated cytotoxicity, ease and speed of performance, and necessity for sophisticated equipment and/or technical skill.

Procedures for separation can include, but are not limited to, physical separation, magnetic separation, using antibody-coated magnetic beads, affinity chromatography, cytotoxic agents joined to a monoclonal antibody or used in conjunction with a monoclonal antibody, including, but not limited to, complement and cytotoxins, and "panning" with antibody attached to a solid matrix, e.g., plate, elutriation or any other convenient technique.

The use of physical separation techniques include, but are not limited to, those based on differences in physical (density gradient centrifugation and counter-flow centrifugal elutriation), cell surface (lectin and antibody affinity), and vital staining properties (mitochondria-binding dye rho 123 and DNA-binding dye Hoechst 33342). These procedures are well known to those of skill in this art.

Techniques providing accurate separation include, but are not limited to, flow cytometry, which can have varying degrees of sophistication, *e.g.*, a plurality of color channels, low angle and obtuse light scattering detecting channels, impedance channels, etc. Cells also can be selected by flow cytometry based on light scatter characteristics, where stem cells are selected based on low side scatter and low to medium forward scatter profiles. Cytospin preparations show the enriched stem cells to have a size between mature lymphoid cells and mature granulocytes.

Alternatively, in a first separation, typically starting with about I x 10⁸⁻⁹, preferably at about 5 x 10⁸⁻⁹ cells, ICD34 can be labeled with a first fluorochrome, while the antibodies for the various dedicated lineages, can be conjugated to a fluorochrome with different and distinguishable spectral characteristics from the first fluorochrome. While each of the lineages can be separated in more than one "separation" step, desirably the lineages are separated at the same time as one is positively selecting for HSCs. The cells can be selected and isolated from dead cells, by employing dyes associated with dead cells (including but not limited to, propidium iodide (PI)). Preferably, the cells are collected in a medium comprising 2% FCS. The particular order of separation is not critical to this invention.

The cells obtained as described above can be used immediately or frozen at liquid nitrogen temperatures and stored for long periods of time, being thawed and capable of being reused. The cells usually will be stored in 10% DMSO, 50% fetal calf serum (FCS), 40% RPMI 1640 medium. Once thawed, the cells can be expanded by use of growth factors and/or stromal cells associated with stem cell proliferation and differentiation.

In a preferred embodiment, before culturing, the HSCs are enriched for cells expressing the cell surface markers CD34 and Thy-1. Preferably, these cells also do not express lineage specific markers associated with specific progenitors. These cells are termed Lin-. Antibodies specific for CD34 and Thy-1 are available and hybridomas can be obtained from the American Tissue Type Collection (ATTC).

The expansion method requires inoculating the population of cells substantially enriched in hematopoietic stem cells and substantially free of stromal cells into an expansion container and in a volume of a suitable medium. Preferably, the cell density is from at least about 1 x 10² cells, preferably 2 x 10³, more preferably 2 x 10⁴ to about 1 x 10⁶ cells/mL of medium, and even more preferably from about 1 x 10⁵ to about 5 x 10⁵ cells/mL of medium, and at an initial oxygen concentration of from about 2 to 20% and preferably less than 8%. In one embodiment. the initial oxygen concentration is in a range from about 4% to about 6%. In one aspect, the inoculating population of cells is derived from bone marrow and is from about 7,000 cells/mL to about 20,000 cells/mL and preferably about 20,000 cell/mL. In a separate aspect, the inoculation population of cells is derived from mobilized peripheral blood and is from about 20,000 cells/mL to about 50,000 cells/mL, preferably 50,000 cells/mL.

Any suitable expansion container, flask, or appropriate tube such as a 24 well plate, 12.5 cm² T flask or gas-permeable bag can be used in the method of this invention. Such culture containers are commercially available from Falcon, Coming or Costar. As used herein, "expansion container" also is intended to include any chamber or container for expanding cells whether or not free standing or incorporated into an expansion apparatus such as the bioreactors described herein. In one embodiment, the expansion container is a reduced volume space of the chamber which is formed by a depressed surface and a plane in which a remaining cell support surface is orientated.

Various media can be used for the expansion of the stem cells. Illustrative media include Dulbecco's MEM, IMDM. X-Vivo 15 (serum-depleted) and RPMI-1640 that can be supplemented with a variety of different nutrients, growth factors, cytokines, etc. The media can be serum free or supplemented with suitable amounts of serum such as fetal calf serum or autologous serum. Preferably, if the expanded cells or cellular products are to be used in human therapy, the medium is serum-free or supplemented with autologous serum. In a preferred embodiment, the medium is X-Vivo 15 (serum-depleted).

As noted above, in one aspect the medium formulations for expansion of HSCs are supplemented with a mpl and FL ligand (preferably TPO and FL) at a concentration from about 0.1 ng/mL to about 500 ng/mL, more usually 10 ng/mL to 100 ng/mL. In addition, IL6, LIF and, optionally, IL3 may be included. As described in the Examples, mimetics of these molecules are also suitable. In another aspect, a c-kit ligand (KL) (also called steel factor (Stl), mast cell growth factor (MGF)) is also added in similar concentrations. As described below, the media formulations for use in the present invention for modifying HSCs are supplemented with a mpl ligand (particularly TPO), a flt3 ligand, fibronectin and/or RetroNectin™ and, optionally, a c-kit ligand, IL-3, IL-6, LIF. Other cytokines which may be added, alone or in combination, are G-CSF, GM-CSF, IL- 1α, IL-11 and MIP-1α. In some aspects, the culture conditions will specifically exclude one or more cytokine, for example IL3.

In one embodiment, the cytokines are contained in the media and replenished by media perfusion. Alternatively, when using a bioreactor system, the cytokines may be added separately, without media perfusion, as a concentrated solution through separate inlet ports. When cytokines are added without perfusion, they will typically be added as a 10X to 100X solution in an amount equal to one-tenth to 1/100 of the volume in the bioreactors with fresh cytokines being added approximately every 2 to 4 days. Further, fresh concentrated cytokines also can be added separately in addition, to cytokines in the perfused media.

### Transduction of Hematopoietic Stem Cell Cultures

The methods of this invention are intended to encompass any method of gene transfer into hematopoietic stem cells, including but not limited to viral mediated gene transfer, liposome mediated transfer, transformation, transfection and transduction, e.g., viral mediated gene transfer such as the use of vectors based on DNA viruses such as adenovirus, adeno-associated virus and herpes virus, as well as retroviral based vectors. The methods are particularly suited for the integration of a nucleic acid contained in a vector or construct lacking a nuclear localizing element or sequence such that the nucleic acid remains in the cytoplasm. In these instances, the nucleic acid or therapeutic gene is able to enter the nucleus during M (mitosis) phase when the nuclear membrane breaks down and the nucleic acid or therapeutic gene gains access to the host cell chromosome. In one embodiment, nucleic acid vectors and constructs having a nuclear localizing element or sequence are specifically excluded.

The HSC cultures described herein are particularly suited for retroviral mediated gene transfer. In retroviral transduction, the transferred sequences are stably integrated into the chromosomal DNA of the target cell. Conditions that favor stable proviral integration include actively cycling cells, as provided for herein.

The terminology for the various stages of the cell cycle are as commonly used and understood in the art. G0 refers to the resting or nongrowing state in the cell cycle; G0 cells are considered noncycling. Cells can be induced to leave the G0 phase and enter into active cycle, i.e. G1, S, G2 and M phases. In culture, this has be achieved by the present invention, for instance, by introducing growth factors into the culture medium.

The HSC cells are transduced with a therapeutic gene. Preferably, the transduction is via a vector such as a retroviral vector. The transduced cells which are obtained by the method of the present invention may be subsequently administered to a recipient. Thus, the invention relates to the treatment of diseases amenable to gene transfer into HSCs.. For example, diseases including, but not limited to, β-thalassemia, sickle cell anemia, adenosine deaminase deficiency, recombinase deficiency, recombinase regulatory gene deficiency, etc. can be corrected by introduction of a therapeutic gene. Other indications of gene therapy are introduction of drug resistance genes to enable normal stem cells to have an advantage and be subject to selective pressure during chemotherapy. Suitable drug resistance genes include, but are not limited to, the gene encoding the multidrug resistance (MDR) protein.

Diseases other than those associated with hematopoietic cells can also be treated by genetic modification, where the disease'is related to the lack of a particular secreted product including, but not limited to, hormones, enzymes, interferons, growth factors, or the like. By employing an appropriate regulatory initiation region, inducible production of the deficient protein can be achieved, so that production of the protein will parallel natural production, even though production will be in a different cell type from the cell type that normally produces such protein. It is also possible to insert a ribozyme, antisense or other message to inhibit particular gene products or susceptibility to diseases, particularly hematolymphotropic diseases.

Retroviral vectors useful in the methods of this invention are produced recombinantly by procedures already taught in the art. For example, WO 94/29438, WO 97/21824 and WO 97/21825 describe the construction of retroviral packaging plasmids and packaging cell lines. As is apparent to the skilled artisan, the retroviral vectors useful in the methods of this invention are capable of infecting HSCs. The techniques used to construct vectors, and transfect and infect cells are widely practiced in the art. Examples of retroviral vectors are those derived from murine, avian or primate retroviruses. Retroviral vectors based on the Moloney (Mo) murine leukemia virus (MuL V) are the most commonly used because of the availability of retroviral variants that efficiently infect human cells. Other suitable vectors include those based on the Gibbon Ape Leukemia Virus (GALV) or HIV.

In producing retroviral vector constructs derived from the Moloney murine leukemia virus (MoMLV), in most cases, the viral gag, pol and env sequences are removed from the virus, creating room for insertion of foreign DNA sequences. Genes encoded by the foreign DNA are usually expressed under the control of the strong viral promoter in the LTR. Such a construct can be packed into viral particles efficiently if the gag, pol and env functions are provided in trans by a packaging cell line. Thus, when the vector construct is introduced into the packaging cell, the gag-pol and env proteins produced by the cell, assemble with the vector RNA to produce infectious virions that are secreted into the culture medium. The virus thus produced can infect and integrate into the DNA of the target cell, but does not produce infectious viral particles since it is lacking essential packaging sequences. Most of the packaging cell lines currently in use have been transfected with separate plasmids, each containing one of the necessary coding sequences, so that multiple recombination events are necessary before a replication competent virus can be produced. Alternatively, the packaging cell line harbors an integrated provirus. The provirus has been crippled so that, although it produces all the proteins required to assemble infectious viruses, its own RNA cannot be packaged into virus. Instead, RNA produced from the recombinant virus is packaged. The virus stock released from the packaging cells thus contains only recombinant virus.

The range of host cells that may be infected by a retrovirus or retroviral vector is determined by the viral envelope protein. The recombinant virus can be used to infect virtually any other cell type recognized by the env protein provided by the packaging cell, resulting in the integration of the viral genome in the transduced cell and the stable production of the foreign gene product. In general, murine ecotropic env of MoMLV allows infection of rodent cells, whereas amphotropic env allows infection of rodent, avian and some primate cells, including human cells. Amphotropic packaging cell lines for use with MoMLV systems are known in the art and commercially available and include, but are not limited to, PA12 and PA317. Miller et al. (1985) *Mol*. *Cell*. *Biol*. **5**:431-437; Miller et al. (1986) *Mol*. *Cell*. *Biol*. 6:2895-2902; and Danos et al. (1988) *Proc. Natl. Acad. Sci. USA* 85:6460-6464. Xenotropic vector systems exist which also allow infection of human cells.

The host range of retroviral vectors has been altered by substituting the env protein of the base virus with that of a second virus. The resulting, "pseudotyped", virus has the host range of the virus donating the envelope protein and expressed by the packaging cell line. Recently, the G-glycoprotein from vesicular stomatitis virus (VSV-G) has been substituted for the MoMLV env protein. Burns et al. (1993) *Proc. Natl*. *Acad. Sci USA* 90:8033-8037; and PCT patent application WO 92/14829. Since infection is not dependent on a specific receptor, VSV-G pseudotyped vectors have a broad host range.

Usually, the vectors will contain at least two heterologous genes or gene sequences: (i) the therapeutic gene to be transferred; and (ii) a marker gene that enables tracking of infected cells. As used herein, "therapeutic gene" can be an entire gene or only the functionally active fragment of the gene capable of compensating for the deficiency in the patient that arises from the defective endogenous gene. Therapeutic gene also encompasses antisense oligonucleotides or genes useful for antisense suppression and ribozymes for ribozyme-mediated therapy. Therapeutic genes that encode dominant inhibitory oligonucleotides and peptides as well as genes that encode regulatory proteins and oligonucleotides also are encompassed by this invention. Generally, gene therapy will involve the transfer of a single therapeutic gene although more than one gene may be necessary for the treatment of particular diseases. In one embodiment, the therapeutic gene is a normal, i.e. wild-type, copy of the defective gene or a functional homolog. In a separate embodiment, the therapeutic gene is a dominant inhibiting mutant of the wild-type. More than one gene can be administered per vector or alternatively, more than one gene can be delivered using several compatible vectors. Depending on the genetic defect, the therapeutic gene can include the regulatory and untranslated sequences. For gene therapy in human patients, the therapeutic gene will generally be of human origin although genes from other closely related species that exhibit high homology and biologically identical or equivalent function in humans may be used, if the gene product does not induce an adverse immune reaction in the recipient. For example, a primate insulin gene whose gene product is capable of converting glucose to glycogen in humans. would be considered a functional equivalent of the human gene. The therapeutic gene suitable for use in treatment will vary with the disease. For example, a suitable therapeutic gene for treating sickle cell anemia is a normal copy of the ϑ-globin gene. A suitable therapeutic gene for treating SCID is the normal ADA gene.

Nucleotide sequences for the therapeutic gene will generally be known in the art or can be obtained from various sequence databases such as GenBank. The therapeutic gene itself will generally be available or can be isolated and cloned using the polymerase chain reaction PCR (Perkin-Elmer) and other standard recombinant techniques. The skilled artisan will readily recognize that any therapeutic gene can be excised as a compatible restriction fragment and placed in a vector in such a manner as to allow proper expression of the therapeutic gene in hematopoietic cells.

A marker gene can be included in the vector for the purpose of monitoring successful transduction and for selection of cells into which the DNA has been integrated, as against cells which have not integrated the DNA construct. Various marker genes include, but are not limited to, antibiotic resistance markers, such as resistance to G418 or hygromycin. Less conveniently, negative selection may be used, including, but not limited to, where the marker is the HSV-tk gene, which will make the cells sensitive to agents such as acyclovir and gancyclovir. Alternatively, selections could be accomplished by employment of a stable cell surface marker to select for transgene expressing stem cells by FACS sorting. The NeoR (neomycin /G418 resistance) gene is commonly used but any convenient marker gene whose sequences are not already present in the recipient cell, can be used.

The viral vector can be modified to incorporate chimeric envelope proteins or nonviral membrane proteins into retroviral particles to improve particle stability and expand the host range or to permit cell type-specific targeting during infection. The production of retroviral vectors that have altered host range is taught, for example, in WO 92/14829 and WO 93/14188. Retroviral vectors that can target specific cell types *in vivo* are also taught, for example, in Kasahara et al. (1994) *Science* **266**:1373-1376. Kasahara et al. describe the construction of a Moloney leukemia virus (MoMLV) having a chimeric envelope protein consisting of human erythropoietin (EPO) fused with the viral envelope protein. This hybrid virus shows tissue tropism for human red blood progenitor cells that bear the receptor for EPO, and is therefore useful in gene therapy of sickle cell anemia and thalassemia. Retroviral vectors capable of specifically targeting infection of HSCs are preferred for in vivo gene therapy.

The viral constructs can be prepared in a variety of conventional ways. Numerous vectors are now available which provide the desired features, such as long terminal repeats, marker genes, and restriction sites, which may be further modified by techniques known in the art. The constructs may encode a signal peptide sequence to ensure that genes encoding cell surface or secreted proteins are properly processed post-translationally and expressed on the cell surface if appropriate. Preferably, the foreign gene(s) is under the control of a cell specific promoter.

Expression of the transferred gene can be controlled in a variety of ways depending on the purpose of gene transfer and the desired effect. Thus, the introduced gene may be put under the control of a promoter that will cause the gene to be expressed constitutively, only under specific physiologic conditions, or in particular cell types.

The retroviral LTR (long terminal repeat) is active in most hematopoietic cells in vivo and will generally be relied upon for transcription of the inserted sequences and their constitutive expression (Ohashi et al. (1992) *Proc. Natl. Acad. Sci.* **89**:11332; Correll et al. (1992) *Blood* **80:**331). Other suitable promoters include the human cytomegalovirus (CMV) immediate early promoter and the U3 region promoter of the Moloney Murine Sarcoma Virus (MMSV), Rous Sarcoma Virus (RSV) or Spleen Focus Forming Virus (SFFV).

Examples of promoters that may be used to cause expression of the introduced sequence in specific cell types include Granzyme A for expression in T-cells and NK cells, the CD34 promoter for expression in stem and progenitor cells, the CD8 promoter for expression in cytotoxic T-cells, and the CD11b promoter for expression in myeloid cells.

Inducible promoters may be used for gene expression under certain physiologic conditions. For example, an electrophile response element may be used to induce expression of a chemoresistance gene in response to electrophilic molecules. The therapeutic benefit may be further increased by targeting the gene product to the appropriate cellular location, for example the nucleus, by attaching the appropriate localizing sequences.

The vector construct is introduced into a packaging cell line which will generate infectious virions. Packaging cell lines capable of generating high titers of replication-defective recombinant viruses are known in the art, see for example, WO 94/29438. Viral particles are harvested from the cell supernatant and purified for in vivo infection using methods known in the art such as by filtration of supernatants 48 hours post transfection. The viral titer is determined by infection of a constant number of appropriate cells (depending on the retrovirus) with titrations of viral supernatants. The transduction efficiency can be assayed 48 hours later by both FACS and Southern blotting.

After viral transduction, the presence of the viral vector in the transduced stem cells or their progeny can be verified by methods such as PCR. PCR can be performed to detect the marker gene or other virally transduced sequences. Generally, periodic blood samples are taken and PCR conveniently performed using e.g. Neo probes if the Neo Resistance gene is used as marker. The presence of virally transduced sequences in bone marrow cells or mature hematopoietic cells is evidence of successful reconstitution by the transduced HSCs. PCR techniques and reagents are well known in the art, See, generally, PCR Protocols, A Guide to Methods and Applications. Innis, Gelfand, Sninsky & White, eds. (Academic Press, Inc., San Diego, 1990) and commercially available (Perkin-Elmer).

The methods provided by the present invention overcome at least 3 deficiencies of conventional protocols for gene transfer in HSCs: culture conditions described herein produce actively cycling HSCs critical for retroviral infection and nucleic acid integration without the concomitant differentiation seen with conventional cytokine treatment or the human toxicity seen with other drugs, as well as increases the number of HSCs available for gene transfer or transplantation.

The populations of cells, culture conditions, cytokines, adhesion molecules and derivatives as described herein may be used for the preparation of medicaments for use in the methods described herein.

The following examples are not intended to limit the present invention in any way.

### EXAMPLES

### ANTIBODIES

To enrich for CD34⁺Thy-1⁺Lin⁻ cells, Tuk3 (anti-CD34 obtained from Dr. A. Ziegler, University of Berlin, Berlin, Germany) was directly conjugated to sulphorhodamine (SR) and GM201 (anti-human Thy-1 from Dr. W. Rettig, Ludwig Cancer Research Institute, New York, NY) was directly conjugated to phycoerythrin (PE) (SyStemix. Palo Alto, CA). FLOPC 21 mouse IgG₃ (Sigma, St. Louis, MO) conjugated to SR (SyStemix) was used as an isotype control for anti-CD34 (Tuk3) staining. Purified mouse IgG₁ (Becton Dickinson, Mountain View, CA) conjugated to PE (SyStemix) was used as a control for anti-Thy-1 staining. The lineage panel of fluorescein isothiocyanate (FITC)-conjugated antibodies Leu-5b (anti-CD2), Leu-M3 (anti-CD14), Leu-M1 (anti-CD15), Leu-IIa (anti-CD16) and SJ25Cl (anti-CD19), FITC-conjugated mouse IgG₁ and IgG₂ₐ PE- and FITC-conjugated BPCA-2 (anti-CD34) as well as PE-conjugated Leu-12 (anti-CD19) and Leu-M9 (antiCD33) were purchased from Becton Dickinson. FITC conjugated antibody D2.10 (anti-glycophorin A) was purchased from AMAC (Westbrook, ME). Hybridomas that produce monoclonal antibodies to monomorphic or polymorphic determinants of HLA molecules were obtained from American Type Culture Collection (ATCC), Rockville, MD.

### Example 1: Enrichment for CD34⁺Thy-1⁺ Lin⁻ cells

*Purification ofCD34*^{*+*}*Thy-1*^{*+*}*Lin*^{*-*} *cells from bone marrow.* Human adult bone marrow (ABM) cells from normal donors were pre-enriched for CD34⁺ cells using a magnetic bead selection device (SyStemix). CD34⁺ cells were also selected from BM from two multi-organ donors and frozen prior to use. CD34⁺ cells were incubated for 10 minutes (min.) on ice with 2 mg/ml heat-inactivated human gamma globulin (Gamunune, Miles Inc., Elkhart, IN) to block non-specific Fc binding. Subsequently the cells were washed with 'staining buffer' (SB). SB contained Hanks Balanced Saline Solution (JRH Biosciences, Lenexa, KS), 0.5% bovine serum albumin (Sigma), 10 mM Hepes (Sigma). Cells were stained for 30 min. on ice with anti-CD34-SR (6 µg/ml), anti-Thy-1-PE (10 µg/ml) and the lineage panel of FITC conjugated antibodies. Appropriate isotype controls were used, as described above. Cells were then washed with SB, and resuspended at a concentration of 10⁶/ml in SB containing 1 µg/ml propidium iodide (PI) (Molecular Probes Inc., Eugene, OR). A Vantage fluorescence activated cell sorter (FACS) (Becton Dickinson Immunocytometry Systems, San Jose, CA) was used to sort live (PI^{lo}) CD34⁺Thy-1⁺Lin⁻ cells. The sorts were reanalyzed to assure clean separation of cell subpopulations.

### Example 2: TPO/FL/KL Effect on Cell Proliferation

*a. PKH26 Fluorescent dye labeling.* Cells were washed with protein-free PBS. The PKH26 dye (Sigma) was diluted 1:250 in the kit diluent. The cell pellet was resuspended at a concentration of 10⁷/ml. This cell suspension was then added to an equal volume of PKH26 and incubated for exactly 4 min. at room temperature (RT). An equal volume of FBS (fetal bovine serum) (Gemini BioProducts, Calabasas, California) was added and incubated for an additional 1 min. at RT. An equal volume of IMDM containing 10% FBS was added. The cells were counted and centrifuged. The pellet was resuspended at a concentration of 10⁵/ml in IMDM/10%FBS with and without cytokines for short term suspension culture. The cells were plated in round-bottom 96-well plates at 100 µl/well.
*b. Short term suspension culture.* PKH26-labeled BM CD34⁺Thy-1⁺Lin⁻ cells were cultured for 6 days at 10⁴ cells/100µl of medium (IMDM, 10% FBS) in round bottom 96-well plates in suspension cultures containing different cytokine combinations. The cytokines used included IL-3 (10 ng/ml), IL-6 (10 ng/ml), LIF (50 ng/ml) (Novartis, Basel, Switzerland), TPO (10-15 ng/ml) (R&D Systems, Minneapolis, MN), KL (50-75 ng/ml) and FL (50-75 ng/ml) (SyStemix). These concentrations were used in the BM studies described herein. Cell numbers. were determined using a hemocytometer and trypan blue to exclude dead cells.
*c*. *Increase of total cell number and of CD34*⁺ *cell number.* The effects of single, double and triple cytokine combinations were studied in 6 day suspension cultures of PKH26-labeled BM CD34⁺Thy-1⁺Lin⁻ cells. Previous studies using PKH26 did not demonstrate a detrimental effect of PKH26 labeling on cellular function.

**Table 1**

| Comparison of fold increase of total cells and CD34⁺ human BM cells in 6 day cultures. | | |
|---|---|---|
| **Cytokines** | **Fold Increase of Total Cells** | **Fold Increase of CD34**^{**+**} **Cells** |
| **TPO** | 0.30 | 0.28 |
| **FL** | 0.72 ± 0.10 | 0.55 ± 0.06 |
| **KL** | 0.67 ± 0.15 | 0.56 ± 0.09 |
| **TPO, KL*** | 1.69 ± 0.47 | 1.02 ± 0.12 |
| **TPO, FL** | 1.67 ± 0.60 | 1.41 ± 0.50 |
| **KL, FL** | 1.67 ± 0.14 | 1.08 ± 0.09 |
| **TPO, KL, FL*** | 4.71 ± 1.50 | 3.43 ± 1.07 |
| **IL-3, TPO, FL** | 3.17 ± 1.10 | 2.28 ± 0.75 |
| **IL-3, IL-6, LIF*** | 0.86 ± 0.10 | 0.68 ± 0.08 |

| | | |
|---|---|---|
| **Footnote to Table 1** * Divided and undivided CD34 subpopulations from these cultures were analyzed in the CAFC assay. The values for TPO, KL, FL represent the means ± SEM of 6 experiments. All other conditions (except TPO (1) and KL (2)) are means ± SEM of 3 experiments. | | |

As shown in Table 1, single cytokines did not increase the number of CD34⁺ or total cells. Combinations of two cytokines out of TPO, KL and FL maintained the CD34⁺ cell number with a slight increase (1.7 fold) in total cell number. Among those tested, the combination of three cytokines TPO, KL and FL induced the highest increases of both total cell (4.7 fold) and of CD34⁺ cell number (3.4 fold). The three-factor combination IL-3, IL-6 and LIF did not stimulate an increase in total cell number.

### Example 3: FACS sorting of CD34/PKH subsets from cultured cells

To determine if PHP numbers were maintained or increased within the population of CD34^{hi} cells which had undergone division, undivided (PKH^{hi}) and divided (PKH^{lo}) subpopulations of CD34^{hi} BM cells were purified from six day cultures containing various cytokine combinations, at concentrations indicated above. The effect of FL alone and in combination with one or two other cytokines was examined in 3-6 experiments (Fig. 1). When cultured in FL alone, most CD34⁺Thy-1⁺Lin⁻ (BM) cells remained undivided PKH26^{hi} (78%). Sixty eight percent (mean 73%) of post-division cells lost CD34 expression. Addition of KL to FL reduced by half the percentage of undivided cells (to 40%), and 55% (mean 58%) of post-division cells lost CD34 expression. Addition of TPO to KL and FL stimulated much greater division (4% remained undivided) with loss of CD34 on only 29% (mean 27%) of post division cells. With other combinations containing TPO e.g. TPO and FL or TPO, IL-3 and FL it was observed that loss of CD34 expression only occurred on about 30% of post-division cells. It has been previously demonstrated that IL-3 induces division, and also differentiation (CD34 loss) of human HSC. (Young et al. (1996), *Blood* **88**:1619). Addition of TPO seemed not only to contribute to greater cell division but also to overcome the effect of IL-3 to promote differentiation.

### Example 4: CAFC Assays

To determine whether retention of CD34^{hi} expression post-division correlated with retention of functional primitive hemopoietic progenitors, PHP, CD34/PKH26 subsets were purified post-culture in three different cytokine conditions and assayed for CAFC activity.
*a. Cobblestone area forming cell (CAFC) assay.* A proportion of the cells were cultured at limiting dilution in the CAFC assay as described previously in Murray et al. (1995) *Blood* 85:368. Briefly, cells were seeded in 96-well plates pre-seeded with a murine stromal cell line (Sys-1) in 1:1 IMDM/RPMI medium (JRH BioSciences, Woodland, CA) containing 1 mM sodium pyruvate (JRH BioSciences) and 5 x 10⁻⁵ M 2-mercaptoethanol (Sigma), 10% FBS, IL-6 and LIF. Limiting dilution ranged from 100 cells per well to 0.78 cells per well. After 5 weeks, wells containing cobblestone areas were enumerated and CAFC frequency of the cell population was calculated using maximum likelihood estimation with SAS software as described in Frzekas de St. Groth (1982) *J. Immunol. Methods* **49**:R11. Statistical significance of CAFC frequency difference. between cultured cell populations was determined by ANOVA. Statistical significance of CAFC number difference between cultured and starting cell populations was determined using the Student t test. Representative wells containing cobblestone areas (at least 10 per sample group) were individually analyzed by FACS for the presence of CD33⁺ immature myeloid, CD19⁺ B lymphoid and CD34⁺ progenitor cell populations in order to estimate the multilineage potential of the original cells.
*b. Analysis of CAFC frequencies and phenotype of cobblestone areas.* The PHP activity of the sorted CD34/PKH26 subpopulations of cultured cells was estimated in vitro by use of the CAFC assay, comparing the CAFC frequencies with the starting population of CD34⁺Thy-1⁺Lin⁻ cells. The mean frequencies of CAFC within the starting population of CD34⁺Thy-1⁺Lin⁻ cells ranged from 1/21 to 1/46 (95% confidence limits 1/16 - 1/52) (Table 2). Due to the limited number of cells obtained from each fresh BM only one cytokine combination could be tested per experiment, giving rise to some tissue variation. In the case of IL-3, IL-6 and LIF, the undivided CD34^{hi}PKH^{hi} subpopulation remained primitive, retaining the same mean CAFC frequency as the pre-culture CD34⁺Thy-1⁺Lin⁻ population. The frequency of CAFC within the small CD34^{hi}PKH^{lo} subpopulation had, however, decreased 21 fold to a mean of 1/440 (1/2491/813).

For the TPO, KL, FL cytokine combination, all cells were PKH^{lo} and these were divided into CD34^{hi} and CD34^{lo/-} subsets, which were placed into the CAFC assay to determine the PHP frequency and multilineage potential of the cells postdivision. In subdividing CD34^{hi} cells based on cell division, the CD34^{lo/-} subpopulation was essentially excluded, because it is known that CAFC are contained mainly within the CD34^{hi} population, as confirmed in Table 2.

**Table 2**

| Mean CAFC frequencies of CD34/PKH26 cell subsets from six day cultures. | | | |
|---|---|---|---|
| **Cytokines** | **Cell Population** | **Day of Culture** | **CAFC Frequency** |
| **IL-3, IL-6, LIF** | CD34⁺Thy-1⁺ | 0 | 1/21 (1/16 - 1/26) |
| | CD34^{hi}PKH^{hi} | 6 | 1/21 (1/15-1/23) |
| | CD34^{hi}PKH^{lo} | 6 | 1/440 (1/249-1/813) |
| **TPO, KL** | CD34⁺Thy-1⁺ | 0 | 1/33 (1/28-1/46) |
| | CD34^{hi}PKH^{hi} | 6 | 1/44 (1/37-1/56) |
| | CD34^{hi}PKH^{lo} | 6 | 1/75 (1/59-1/89) |
| **TPO, KL, FL** | CD34⁺Thy-1⁺ | 0 | 1/46 (1/41 - 1/52) |
| | CD34^{hi}PKH^{hi}* | 6 | |
| | CD34^{hi}PKH^{lo} | 6 | 1/42 (1/33 - 1/59) |
| | CD34^{lo/-}PKH^{lo} | 6 | 1/2898 (1/1743-1/13415) |

| | | | |
|---|---|---|---|
| **Footnote to Table 2** CAFC frequencies at 5 weeks were calculated using maximum likelihood estimation with SAS software. The 95% confidence limits are shown in parentheses. Mean of 6 experiments for TPO, KL, FL and a mean of 2 experiments for the other cytokine combinations. * Not determined because all cells had undergone division after 6 days in culture in TPO, KL, FL. | | | |

The majority of cells cultured in IL-3, IL-6 and LIF did not divide (mean 75%) by day 6 and, therefore, we sorted CD34^{hi}PKH^{hi} versus CD34^{hi}PKH^{lo} (mean 7.5%) (Table 2). The same cell populations were sorted from cultures with TPO and KL in which a mean of 53% of cells remained undivided and a mean of 23% of cells were CD34^{hi} PKH^{lo}. In TPO, KL and FL all the cells had divided and therefore we sorted for CD34^{hi} PKH^{lo} (mean 71%) versus the CD34^{lo/-}PKH^{lo} (mean 26%) population of differentiated post-division cells.

**Table 3**

| B lymphoid Potential and CD34⁺ progenitor cells in long term stromal cultures | | | | |
|---|---|---|---|---|
| **Cytokines** | **Cell Population** | **Day of Culture** | **Percent of Positive Wells** | |
| | | | **CD19**^{**+**} **B lymphoid** | **CD34**^{**+**} **progenitors** |
| **IL-3, IL-6, LIF** | CD34⁺Thy-1⁺ | 0 | 71.8 ± 8.2 | 63.2 ± 26.8 |
| | CD34^{hi}PKH^{hi} | 6 | 35.9 ± 2.6 | 40.4 ± 9.6 |
| | CD34^{hi}PKH^{lo} | 6 | 0 | 0 |
| **TPO, KL** | CD34⁺Thy-1⁺ | 0 | 58.2 ± 19.7 | 79.1 ± 9.9 |
| | CD34^{hi}PKH^{ho} | 6 | 71.8 ± 5.1 | 28.2 ± 5.1 |
| | CD34^{hi}PKH^{lo} | 6 | 63.4 ± 0.9 | 3.6 ± 3.6 |
| **TPO, KL, FL** | CD34⁺Thy-1⁺ | 0 | 41.5 ± 16.5 | 66.9 ± 16.8 |
| | CD34^{hi}PKH^{lo} | 6 | 54.2 ± 8.5 | 49.0 ± 27.1 |
| | CD34^{lo/-}PKH^{lo} | 6 | ND | ND |
| **Footnote to Table 3** Wells were scored positive if >1% of cells were positive for the surface marker. All cobblestone areas analyzed contained CD33+ myeloid cells. Values are the means ± SEM for 2 experiments (IL-3, IL-6, LIF and TPO, IL) or 6 experiments (TPO, KL, FL). ND means not determined due to no or limited number of wells containing cobblestone areas. | | | | |

After culture with TPO and KL, again the undivided CD34^{hi}PKH^{hi} cells had a similar CAFC frequency to the uncultured CD34⁺Thy-1⁺Lin⁻ population. In these conditions the mean frequency of CAFC in the divided CD34^{hi}PKH^{lo} subpopulation was reduced 2.3 fold, compared with the starting cell population (Table 2). CD34^{hi}PKH^{lo} cells retained the potential, at limiting dilution, to give rise to CD19⁺ B lymphoid, CD33⁺ myeloid and CD34⁺ progenitor cells after 5 weeks of culture in the CAFC assay. The proportion of wells containing >1 % CD34⁺ cells was, however, reduced 22 fold compared to the starting cell population (Table 3). For each cell population, all cobblestone areas analyzed contained CD33⁺ myeloid cells.

The mean values for 6 experiments with the combination of TPO, KL and FL are shown in Table 3. The mean CAFC frequency remained the same in the CD34^{hi}PKH^{lo} subpopulation, compared to the starting CD34^{hi}PKH^{lo} cell population. These cells also, at limiting dilution, retained their ability to give rise to CD19⁺ B lymphoid progenitors, CD33⁺ myeloid cells and CD34⁺ progenitor cells. CD19⁺ cells were observed in a similar proportion (about 50%) of cobblestone areas examined for both the uncultured CD34⁺Thy-1⁺Lin⁻ cells and post-culture CD34^{hi}PKH^{lo} cells. Forty nine percent of cobblestone areas generated from CD34^{hi}PKH^{lo} cells contained CD34⁺ cells, as compared to 67% for the uncultured CD34⁺Thy-1⁺Lin⁻ cells (Table 3). As expected, the CD34^{lo/}-PKH^{lo} cells had very low CAFC frequency (mean of 1/3000). Analysis of a minimum of 10 small cobblestone areas generated from this population showed that divided CD34^{hi} cells from cultures containing IL-3, IL-6 and LIF gave rise to only CD33⁺ myeloid cells (Table 3).

*Increase in CAFC numbers among total and CD34*^{*hi*}*PKH*^{*lo*} *cells.* We compared the increase of CAFC numbers from CD34⁺Thy-1⁺Lin⁻ cells in different culture conditions (Fig. 2). Of the 3 different cytokine combinations analyzed, only TPO, KL and FL increased the mean number of total cells, CD34⁺ cells and CAFC (Table 1 and Fig 2). When the number of CAFC within the CD34^{hi}PKH^{lo} population is compared to the original number of CAFC placed in culture, only the TPO, KL and FL had CAFC numbers that increased among divided cells (mean 3.2 fold), although values ranged from maintenance to a 7.6 fold increase. The CAFC number among divided CD34^{hi} cells at day 6 was not significantly different from the number measured among CD34+Thy-1⁺Lin⁻ cells at day 0 for TPO and KL cultures (n = 2, p = 0.19), but increased CAFC number among CD34^{hi}PKH^{lo} cells in TPO, KL and FL cultures approached statistical significance (n = 6, p = 0.07). The number of measurable CAFC among divided CD34^{hi} cells from IL-3, IL-6 and LIF cultures had significantly decreased (n = 2, p = 0.03). All CAFC detectable in D6 IL-3, IL-6 and LIF cultures, were derived from undivided CD34^{hi} cells.

### Example 5: SCID-hu bone assay

CD34^{hi}PKH26^{lo} and CD34^{lo}PKH26^{lo} subsets from TPO, KL and FL cultures were assayed for in vivo SCID-hu bone repopulating activity.
*a. SCID-hu bone assay.* The SCID-hu bone assay was performed as previously described in Murray et al. (1995) *Blood* 85:368 and Chen et al. (1994) *Blood* **84**:2497. C.B-17 scid/scid mice were used as recipients of human fetal bone grafts. First, limiting dilution analysis was performed to determine the dose of CD34⁺Thy-1⁺Lin⁻ cells which reliably gives donor reconstitution in the SCID-hu bone model. HLA-mismatched fetal bone grafts were injected with cell doses ranging from 1000-30,000 CD34⁺Thy-1⁺Lin⁻ cells per graft into mice which received whole body irradiation (400 md) shortly before cell injection. To achieve a sufficient number of grafts at each dose, four tissue donors were used in four separate experiments. Eight weeks after injection, the bone grafts were recovered and the bone marrow cells harvested and analyzed for donor cell engraftment using FITC conjugates of allotype-specific HLA antibodies versus PE-conjugated anti-CD 19, anti-CD33 and anti-CD34. Total human cells were detected with W6/32-PE (anti-human HLA class I MHC molecule monomorphic determinant). Cells were analyzed on a FACScan analyzer (Becton Dickinson Immunocytometry Systems). Grafts having at least 1% of hematopoietic cells bearing donor HLA antigen were considered positive. The percentage of grafts showing donor reconstitution was assayed for each cell dose tested. At five times the limit dose, or 10,000 cells, donor reconstitution was observed in all grafts.
   Uncultured BM CD34⁺Thy-1⁺Lin⁻ as well as CD34^{hi}, PKH^{lo} and CD34^{lo/-} PKH^{lo} cells from D6 cultures in TPO, KL and FL were sorted and injected (10,000 cells per graft) into SCID-hu bone grafts. Eight weeks after injection, the bone grafts were analyzed for engraftment of donor CD33⁺, CD19⁺ and CD34⁺ cells.
*b. Dose of uncultured CD34*^{*+*}*Thy-1*^{*+*}*Lin*^{*-*} *cells which gives reconstitution of 100% of SCID-hu bone grafts.* The percentage of grafts showing donor reconstitution at each CD34⁺Thy-1⁺Lin⁻ cell dose tested is shown in Fig 4. By Poisson distribution analysis, the frequency of SCID-hu bone repopulating cells was 1 per 2000 CD34⁺Thy-1⁺Lin⁻ cells. At five times this limit dose or 10,000 cells, donor reconstitution was observed in 100% of grafts.
*c. Engraftment of CD34*^{*hi*}*PKH*^{*lo*} *cells from 6 day culture in TPO, KL and FL in SCID-hu bone.* CD34^{hi}PKH^{lo} cells from 6 day cultures of CD34⁺Thy-1⁺Lin⁻ cells in TPO, KL and FL contained increased numbers of CAFC. In addition, we asked whether the same cell population retained its ability to repopulate human bone in vivo, using the SCID-hu bone assay, as described for example in Murray et al. (1995), *supra* and Chen et al. (1994), *supra*. In order to obtain sufficient cells, CD34⁺Thy-1⁺Lin⁻ cells were purified from cryopreserved BM CD34' cells isolated from multi-organ donors. Uncultured CD34⁺Thy-1⁺Lin⁻ cells and CD34^{hi}PKH^{lo} from D6 TPO, KL and FL cultures were injected into the fetal human bone grafts. Ten thousand cells were injected per graft, since this cell dose provides consistent engraftment of uncultured BM CD34⁺Thy-1⁺Lin⁻ cells (Fig 3).

Cultured CD34^{hi}PKH^{lo} cells engrafted to a similar level as the uncultured population of CD34⁺Thy-1⁺Lin⁻ cells (4/4 grafts) (Fig 4 and Table 4). In experiment A (Table 4), the mean percentage of donor cells was 34.3 ± 22.3% for CD34^{hi}PKH^{lo} cells, comparable with 25.0 ± 13.5% for uncultured CD34⁺Thy-1⁺Lin⁻ cells. FACScan analysis shows that multilineage engraftment occurred in both cases, since the cells isolated from the bones after 8 weeks included donor B lymphoid (CD19⁺), myeloid (CD33⁺) and progenitor cells (CD34⁺) (Fig 4).

In a second experiment (B), 4/4 grafts injected with CD34^{hi}PKH^{lo} cells from D6 TPO, KL and FL cultures showed multilineage engraftment, with a mean of 59.0 ± 12.0% donor cells (Table 4). The results confirm that after 6 days of culture, in vivo marrow repopulating capacity of CD34⁺Thy-1⁺Lin⁻ cells is retained within the CD34^{hi} population postdivision in TPO, KL and FL.

**Table 4**

| CD34+ cells which have divided during 6 days culture in TPO, KL and FL retain their capacity for marrow repopulation in vivo in the SCID-hu bone assay | | | | | | | |
|---|---|---|---|---|---|---|---|
| **Cell Population** | **Exp** | **CAFC Freq.** | **Pos. Graft** | **% Donor** | **% CD19**^{**+**} | **% CD33**^{**+**} | **% CD34**^{**+**} |
| **CD34**^{**hi**}**Thy-1**^{**+**} | A | 1/106 | 4/4 | 25.0 ± 13.5 | 22.0 ± 12.5 | 4.8 ± 2.8 | 4.5 ± 0.5 |
| | B | 1/38 | ND* | ND | ND | ND | ND |
| **CD34**^{**hi**}**PKH**^{**lo**} | A | 1/36 | 4/4 | 34.3 ± 22.3 | 31.3 ± 19.8 | 6.7 ± 6.2 | 3.9 ± 3.4 |
| | B | 1/26 | 4/4 | 59.0 ± 12.0 | 58.0 ± 11.5 | 1.9 ± 0.6 | 5.0 ± 1.0 |
| **CD34**^{**lo/**}**-PKH**^{**lo**} | A | 1 /6600> | 1/4 | 3.5 ± 3.5 | 3.3 ± 4.9 | 1.3 ± 1.8 | ND |
| | B | 1 /932 | 0/4 | 0 | 0 | 0 | ND |

| | | | | | | | |
|---|---|---|---|---|---|---|---|
| **Footnote to Table 4** 10,000 cells were injected per bone graft. Errors shown are SEM. Grafts were analyzed 8 weeks following injection for donor cells expressing the HLA market of the injected cells. ND is not determined * Mice used for injection of uncultured CD34⁺Thy-1⁺Lin⁻ cells in experiment B died prior to analysis of the grafts. > No CAFC detected among 6600 cells plated. | | | | | | | |

### Example 6: MPB CD34⁺ cells cultured for 90 hours in different cytokine cultures:

a. *Cell culture and cytokines.* Cells were counted, resuspended and cultured for 90 hours at 2 x 10⁵ cells per ml in serum-free X-Vivo 15 medium (BioWhittaker, Walkersville, MD) containing glutamine (JRH BioSciences, Lenexa, KS) and 1% bovine serum albumin (BSA) (Sigma, St. Louis, MO) in wells of 24 well flat bottom plates (Corning Costar Corp., Cambridge, MA) at 37°C. The cultures contained different cytokines combinations. Cytokines used included recombinant human IL3 (20 ng/ml), IL6 (20 ng/ml), LIF (100 ng/ml) (Novartis Pharmaceuticals Corp., Basel, Switzerland), TPO (50-100 ng/ml) (R&D Systems, Minneapolis, MN), KL (100 ng/ml) and FL (100 ng/ml) (SyStemix, Palo Alto, CA).
   Before culturing, a sample of MPB CD34⁺ cells was incubated with 1 mg/ml heat-inactivated human gamma-globulin (Gamimune, Miles Inc. Elkhart, IN) for 10 minutes (min) on ice. Cells were then stained with anti-CD34-FITC (HPCA-2-FITC, Becton Dickinson) and anti-Thy-1-Cy5 (PR13, Systemix) or with appropriate isotype controls IgG₁-FITC (Becton Dickinson, San Jose, CA) and IgG₁-Cy5 (Systemix, Palo Alto, CA) for 30 min on ice. Cells were washed and resuspended in PBS (JRH BioSciences, Lenexa, KS), 2% fetal calf serum (FCS) (Hyclone, Logan, Utah) and analyzed on a FACS-Calibur Flow Cytometer (Becton Dickinson).
   At the end of the culture period, cells were harvested and viable cell numbers were determined using trypan blue exclusion. Cells were stained with anti-CD34-Cy5 (PR20, SyStemix) and anti-Thy-1-PE (PR13, SyStemix) or the appropriate isotype controls IgG₁-Cy5 and IgG₁-PE (SyStemix) before analysis on a FACS-Calibur flow cytometer. Cells with high propidium iodide content were excluded from the analysis.
*b*. *Increase in number of total cells and of the CD34*^{*+*}*Thy-1*^{*+*} *cell subset*. The percentages of total CD34⁺ progenitors and the more primitive CD34⁺Thy-1⁺ subset were measured. The mean data from 3-12 different MPB donors are shown in Figure 5. Beginning with > 90% pure CD34⁺ selected cells, 89% of cells expressed CD34 at the end of culture with addition of TPO alone, compared to 79% with KL or FL alone. There was no significant difference in the percentage of CD34⁺Thy-1⁺ cells detectable, which was 13-14% with each of these single cytokines. Although both combinations of TPO, FL and TPO, KL preserved 91-92% of CD34⁺ cells, a mean of 20.7 +/- 5.2% CD34⁺Thy-1⁺ cells were detected in TPO and FL compared to only 5.5 +/- 3% in TPO and KL (*P* = 0.0084). The percentage of CD34⁺Thy-1⁺ cells was also higher (mean 20.7%) in TPO and FL than in the three factor combination of TPO, FL, KL (mean 6.8%) (P < 0.0001). Preservation of this primitive phenotype was significantly lower in IL-3, IL-6 and LIF (mean 3.7%) than in TPO, FL, KL (P = 0.02).
   The fold increase of CD34⁺Thy-1⁺ cell number is shown in Figure 6. individually, TPO, FL and KL each failed to increase the number of CD34⁺Thy-1⁺ cells. Cultures including TPO and FL or TPO and KL in combination gave the highest increase of CD34⁺Thy-1⁺ cell number. Considering the level of variation among the MPB samples, there was no significant difference among such cultures, which all resulted in maintenance or a small increase (1.2 - 1.5 fold) of CD34⁺Thy-1⁺ cell number.
*c. CAFC Assays in Mobilized Peripheral Blood Cells.* CAFC assays were also conducted on mobilized peripheral blood cells. CD34⁺Thy-1⁺ cells were purified from mobilized peripheral blood cells using flow cytometry and were cultured at 4x10e5 cells/mL in X-VIVO 15, 1% BSA and the cytokines indicated in Figure 7. After five days in culture, the cells were analyzed for Thy-1 expression by flow cytometry, and CAFC activity as described above. Fold change in total CAFC numbers in the post-culture Thy-1⁺ populations relative to the started population are shown Figure 7 as the mean of four experiments.
*d. CFSE dye labeling.* Mobilized peripheral blood (MPB) CD34⁺-selected cells were labeled with carboxyfluorescein-diacetate succinimidylester CFSE dye (Molecular Probes Inc., Eugene, OR) at a cell concentration of 3x10⁶/mL and a dye concentration of 1.25µM in Iscove's modified Dulbecco's medium (IMDM) without phenol red (JRH Biosciences) in the dark, at room temperature for 10 min, with occasional mixing. The labeling was stopped by the addition of 1/5 volume of FCS. Ten ml of X-Vivo 15 medium containing 1% BSA were then added to the cells, which were centrifuged at 400g for 10 min.
*e. Effect of TPO mimetics on HSC replication.* The effect of a TPO mimetic on cell expansion was also tested. The TPO mimetic used is known as peptide AF13948, having the amino acid sequence shown in Cwirla et al. (1997) *supra.* Mobilized peripheral blood CD34⁺ cells were labeled with 1.25 µM CFSE dye, which allows cell division to be measured fluorimetrically. The cells were then cultured at 2x10⁵ cells/mL in X-VIVO 15, 1 % BSA and the cytokines indicated in Table 5. After 4 days of culture, the cells were analyzed for Thy-1 expression and cell division by flow cytometry. Fold expansion of total cell numbers and viability were determined by hemocytometer counting of trypan blue stained samples. Results are summarized in Table 5.

**Table 5**

| Cell Culture and Expansion and Viability | | | | |
|---|---|---|---|---|
| **Cytokine** | **Expansion** | **% Viable*** | **% Divided** | **% Thy-1+** |
| KL (100) | 0.33 | 28.4 | 24.82 | 1.57 |
| TPO (100) | 0.65 | 57 | 66.2 | 4.97 |
| MTPO (50) | .079 | 52 | 62.8 | 6.18 |
| KL, TPO (100, 100) | 1.81 | 87.3 | 86.5 | 7.42 |
| KL, MTPO (100, 50) | 1.84 | 89.3 | 87.3 | 8.6 |
| KL, MTPO (100, 25) | 1.51 | 85 | 87.3 | 8.59 |
| KL, MTPO (100, 10) | 1.55 | 82.4 | 86.8 | 11.15 |
| KL, MTPO (100, 1) | 1.07 | 72.9 | 81.4 | 16.36 |
| KL, MTPO (100, 0.1) | 0.93 | 64.6 | 68.5 | 9.54 |
| KL, MTPO (100, 0.01) | 0.43 | 41.4 | 45.5 | 14.2 |
| KL, MTPO (100, 0.001) | 0.30 | 29.7 | 31.2 | 4.58 |

| | | | | |
|---|---|---|---|---|
| * Percent viability measured by trypan blue KL= c-kit ligand TPO=thrombopoietin MTPO=thrombopoietin mimetic | | | | |

### Example 7: Retention of Stem Cell Function Among CD34⁺Thy-1⁺ Cells after Each Cell Division

CFSE labeled CD34⁺ MPB cells as described herein above were cultured in X-Vivo 15 medium containing 1% BSA supplemented with combinations of TPO (50ng/ml) FL (100ng/ml), KL (100 ng/ml) and IL6 (20 ng/ml) for approximately 112 hours at 2 x 10⁵ cells/mL in 24 well flat bottom plates at 37 °C in a humidified incubator.

Cultured cells were harvested, enumerated and stained with anti-CD34-Cy5 and anti-Thy-1-PE or the appropriate isotope controls as described above. CD34⁺ Thy-1⁺ populations were isolated by flow cytometry as described above for sorting cells. Sort regions for discreet numbers of cell divisions (FITC fluorescence) among Thy-1⁺ cells were established based on paraformaldehyde fixed undivided control populations and a visibly diminished event density between each division. The CD34⁺Thy-1⁺ cells cultured in TPO, FL and KL expanded about two-fold in total number. The CD34⁺ Thy-1⁺ cells cultured in TPO, FL and IL6 maintained starting cell numbers (data not shown). The percentage of cells expressing Thy- 1 were slightly higher in TPO, Fl, IL6 than in TPO, FL, KL (27.4% vs. 20.6%); however, the TPO FL, IL6 cultures maintained fewer total Thy-1 expressing cells (0.34 vs. 0.43 of starting cell number). These differences were not statistically significant (data not shown).

Cultured cell populations having undergone 1, 2, 3, or 4 divisions were purified by flow cytometry. Only cells still expressing Thy-1 post-culture were tested, since the majority of HSC activity resided in this population. Thy-1⁺cells cultured in TPO, FL. KL or TPO, FL, IL6 at 100ng/ml TPO, 100ng/ml FL, 100ng/ml KL and 20ng/ml IL6, retained CAFC activity relative to fresh CD34⁺Thy-1⁺ cells after at least two divisions. CAFC activity decreased after 4 division for TPO, FL, KL or 3 divisions TPO, FL, IL6. (data not shown) Engraftment in SCID-hu bone was observed at all four divisions for both cytokine combinations when either 5,000 or 15,000 cells were injected per graft (Fig. 8). Numbers of grafts containing human cells, out of the total number injected (engraftment rate), did not change significantly between uncultured CD34⁺ cells or cultured CD34⁺ Thy-1⁺ cells having undergone various numbers of divisions in either cytokine combination. The average number of donor cells recovered from the grafts injected with TPO, FL, KL cultured cells was approximately 2 fold higher than grafts injected with TPO, FL, IL6 cultured cells, and was close to the number of donor cells obtained from grafts injected with uncultured CD34⁺ cells. It was concluded that CD34⁺Thy-1⁺ cells cultured in TPO, FL, KL or TPO, FL, IL6 can undergo up to four divisions without losing apparent HSC function on a per cell basis.

### Example 8: Gene Transfer into Cultured Hematopoietic Cells

Hematopoietic stem cells cultured as described above in Examples 1 - 7 with 50 - 100ng/ml TPO, 100ng/ml KL, 100ng/ml FL, 20ng/ml IL3, 20ng.ml IL6 and/or 100 ng/ml LIF were infected with either the (1) L Mily vector (which expresses Lyt2) or (2) the LMTNL vector (to analyze rev gene marking by PCR). The cells were infected by adding the appropriate vector to the culture medium. As shown below in Tables 6-8, TPO, KL and FL give 4.9 fold higher gene marking of LTC-CFC; 5 fold higher Lyt2 expression among post SyS1 CD34+ progenitor cells; and 4.7 fold higher Lyt2 expression among post SyS1 CD14+ monocytes compared to cells cultured in IL-3, IL-6 and LIF.

**Table 6**

| Percentage of HSCs marked by rev | |
|---|---|
| **CYTOKINES IN CULTURE** | **% CELLS REV MARKING In 5- WEEK SyS1 CULTURE (mean 3 - 4 experiments)** |
| TPO, KL and FL | 73.8% |
| TPO, FL and IL-6 | 63.2% |
| IL-3, IL-6 and LIF (control) | 25.1% |

**Table 7**

| Percentage of CD34⁺ cells expressing Lyt2 transgene | |
|---|---|
| **CYTOKINES IN CULTURE** | **% CD34+ CELLS EXPRESSING LYT2 TRANSGENE in SyS1 CULTURE (mean 2-4 experiments)** |
| TPO, KL and FL | 3.8% |
| IL-3, IL-6, LIF, FL and TPO | 1.9% |
| TPO, FL and IL-6 | 1.6% |
| IL-3, IL-6 and LIF (control) | 0.75% |

**Table 8**

| Percentage of CD14⁺ monocytes expressing Lyt2 transgene | |
|---|---|
| **CYTOKINES IN CULTURE** | **% CD14+ CELLS EXPRESSING LYT2 TRANSGENE in SyS1 CULTURE (mean 2-4 experiments)** |
| TPO, KL and FL | 8.0% |
| IL-3, IL-6, LIF, FL and TPO | 2.8% |
| TPO, FL and IL-6 | 2.4% |
| IL-3, IL-6 and LIF (control) | 0.3% |

Polymerase chain reaction (PCR) was also conducted to determine gene transfer into HSCs cultured in various cytokine combinations. Table 9 summarizes the percentage of colonies and grafts expressing the transgene.

**Table 9**

| **Cytokine Combination** | **Results** | | | |
|---|---|---|---|---|
| | **LTC-CFC** | | **SCID-HU Mouse** | |
| | **REV+/ β-globin+** | **%REV+ colonies** | **REV+/ β-globin+ grafts** | **%REV+ grafts** |
| IL3, IL6, LIF | 11/53 | 20.8 | 2/6 | 33.4 |
| TPO, FL, IL6 | 28/36 | 77.8 | 4/6 | 66.7 |
| TPO, FL, KL | 45/49 | 91.8 | 3/6 | 50.0 |
| TPO, FL, IL6, LIF | 20/51 | 39.2 | ND | ND |
| TPO, FL, IL3, IL6, LIF | 46/58 | 79.2 | ND | ND |
| | | | | |
| IL3, IL6, LIF | 3/46 | 6.5 | 3/6 | 50.0 |
| TPO. FL, IL6 | 12/48 | 25.0 | 3/6 | 50.1 |
| TPO, FL, KL | 24/57 | 42.1 | 2/6 | 33.4 |
| | | | | |
| IL3, IL6, LIF | ND | ND | 0/4 | 0.0 |
| TPO, FL, IL6 | ND | ND | 0/4 | 0.0 |
| TPO, FL, KL | ND | ND | 0/4 | 0.0 |
| | | | | |
| TPO, FL, IL3, LIF | ND | ND | 2/2 | 100.0 |
| TPO, FL, IL6, LIF | ND | ND | 2/2 | 100.0 |
| TPO, FL, IL3, IL6, LIF | ND | ND | 3/4 | 75.0 |
| | | | | |
| TPO, FL, IL3, LIF | 33/50 | 66.0 | 3/3 | 100.0 |
| TPO, FL, IL6, LIF | 49/55 | 89.5 | 1/3 | 33.4 |
| TPO, FL, IL3, IL6, LIF | 48/56 | 86.9 | 4/4 | 100.0 |
| | | | | |
| TPO, FL, IL3, LIF | 31/51 | 60.8 | ND | ND |
| TPO, FL, IL6, LIF | 22/50 | 44.0 | ND | ND |
| TPO, FL, IL3, IL6, LIF | 34/53 | 64.2 | ND | ND |
| | | | | |
| FL, IL3, IL6, LIF | ND | ND | ND | ND |
| TPO, IL3, IL6, LIF | ND | ND | ND | ND |
| TPO, FL, IL3, IL6, LIF | ND | ND | ND | ND |
| TPO, FL, IL6, LIF | ND | ND | ND | ND |

### Example 9 : Genetic Modification of HSCs in Presence of Fibronectin

To determine the potential for RetroNectin™ (Takara Shuzo Co. Ltd., Otsu Shigi, Japan) to enhance transgene expression in the progeny of transduced HSC, CD34⁺ selected cells, transduced with the ProPak-based PP-A.LMiLy vector in the presence and absence of RetroNectin™ and a variety of cytokines, were incubated long-term on preformed Sysl monolayers. After five weeks, the human cells in the cultures were stained for FACs analysis of the cell surface expression for CD34 and Lyt2. The results of representative experiments are shown in Figure 9 and Table 10. The data indicates that the transgene expression (Lyt2 expression) on CD34⁺ cells that are maintained in the long-term Sys1 culture is significantly enhanced by transducing cells on RetroNectin™ coated plates in the presence of a cytokine cocktail comprising IL3, IL6, LIF, FL and TPO. Cytokine concentrations are described above in Example 8. As shown in Figure 9, Lyt2 expression was 3.7% in the populations transduced with IL3, IL6, LIF, FL and TPO without RetroNectin™ compared to 15.2% in the populations transduced on RetroNectin™ coated plates in the presence of the same cytokines. The use of RetroNectin™ showed an approximate 4-fold enhancement. In the absence of TPO, no enhancement was seen. There was no effect on the percent of cells in the long-term cultures that retained the CD34 phenotype whether transduced with the different cytokine cocktails in the presence or absence of RetroNectin™.

**Table 10**

| Gene Transfer: MPB-CD34⁺ Cells in Various Cytokine Combinations. Transduction Efficiency (%Rev⁺) of Clonogenic Cells | | | | |
|---|---|---|---|---|
| | | **IL3, IL6, LIF** | **IL3, IL6, LIF, FL** | **IL3, IL6, FL, TPO** |
| **CFU-C** | **Mean** | **85.1** | **89.9** | **86.0** |
| | Std. D. | 11.5 | 8.4 | 10.9 |
| | Range | (60-93.3) | (75.8-98.8) | (68.8-98) |
| | n= | 9 | 9 | 6 |
| **LTC-CFC** | **Mean** | **14.1** | **46.9** | **67.3** |
| | Std. D. | 10.2 | 25.7 | 26.4 |
| | Range | (3.9-35) | (14-85) | (28-94) |
| | n= | 10 | 10 | 7 |
| RetroNectin™ Protocol | | | | |

## Claims

1. A method for modifying a hematopoietic stem cell, comprising contacting a gene delivery vehicle comprising a polynucleotide sequence with a population of hematopoietic stem cells cultured with fibronectin or RetroNectin™ and in the presence of an amount of an mpl ligand and a flt3 ligand (FL) which is sufficient to promote the survival, expansion and/or transduction of the hematopoietic stem cells, wherein: (i) the method is not a method for treatment of the human or animal body by surgery or therapy; and (ii) the method does not make use of human embryos for industrial or commercial purposes.

2. The method of claim 1, further comprising culturing the hematopoietic stem cells in the presence of a c-kit ligand.

3. The method of claim 2, further comprising culturing the hematopoietic stem cells in the presence of interleukin 3 (IL3).

4. The method of any one of claims 1 to 3, wherein the polynucleotide sequence encodes a product selected from the group consisting of a peptide, a ribozyme and an antisense sequence.

5. The method of any one of claims 1 to 4, wherein the gene delivery vehicle is selected from the group consisting of a retroviral vector, a DNA vector and a liposomal delivery vehicle.

6. A method for modifying a hematopoietic stem cell, comprising contacting a gene delivery vehicle comprising a polynucleotide sequence with a population of hematopoietic stem cells cultured with fibronectin or RetroNectin^{TM} and in the presence of an amount of thrombopoietin (TPO), a flt3 ligand (FL), and interleukin 6 (IL6), which is sufficient to promote the survival, expansion and/or transduction of the hematopoietic stem cells, wherein: (i) the method is not a method for treatment of the human or animal body by surgery or therapy; and (ii) the method does not of human embryos for industrial or commercial purposes.

7. The method of claim 6, further comprising culturing the stem cells in the presence of leukemia inhibitory factor (LIF).

8. The method of claim 6, further comprising culturing the stem cells in the presence of interleukin 3 (IL3).

9. The method of claim 6, further comprising culturing the stem cells in the presence of a c-kit ligand.

10. The method of claim 8, further comprising culturing the stem cells in the presence of a c-kit ligand.

11. The method of claim 6, wherein the polynucleotide sequence encodes a product selected from the group consisting of a peptide, a ribozyme and an antisense sequence.

12. The method of claim 6, wherein the gene delivery vehicle is selected from the group consisting of a retroviral vector, a DNA vector and a liposomal delivery vehicle.

13. The method of any of claims 1 to 12, further comprising the expansion of hematopoietic stem cells in culture.

14. The method of claim 13, further comprising culturing the cells with interleukin 3 (IL3).

15. The method of claim 13, further comprising culturing the cells with leukemia inhibitory factor (LIF).

16. The method of claim 13, further comprising culturing the cells with a c-kit ligand.

17. The method of claim 13, wherein the hematopoietic stem cells are **characterized by** the capability of self-renewal and the ability to give rise to all hematopoietic cell lineages.

18. The method of claim 13, wherein the hematopoietic stem cells are human hematopoietic stem cells.

19. The method of claim 18, wherein the human hematopoietic stem cells are CD34⁺.

20. The method of claim 18, wherein the human hematopoietic stem cells are CD34⁺Thy-1⁺.

21. The method of claim 18, wherein the human hematopoietic stem cells are CD34⁺Thy-1⁺Lin.

## Patentansprüche

1. Verfahren zum Modifizieren einer hematopoietischen Stammzelle, umfassend das in Kontakt bringen eines Gen-Übermittlungs-Vehikels, umfassend eine Polynucleotidsequenz, mit einer Population an hematopoietischen Stammzellen, die mit Fibronectin oder RetroNectin™ und in Anwesenheit einer Menge an mpl-Ligand und flt3-Ligand (FL) kultiviert werden, welche ausreicht, das Überleben, die Expansion und/oder die Transduktion der hematopoietischen Stammzellen zu fördern, worin: (i) das Verfahren keine Verfahren zur Behandlung des menschlichen oder tierischen Körpers mittels Operation oder keine Therapie ist, und (ii) das Verfahren humanen Embryos nicht für industrielle oder kommerzielle Zwecke verwendet.

2. Verfahren nach Anspruch 1, zusätzlich umfassend das Kultivieren der hematopoietischen Stammzellen in Anwesenheit eines c-kit Liganden.

3. Verfahren nach Anspruch 2, zusätzlich umfassend das Kultivieren der hematopietischen Stammzellen in Anwesenheit von Interleukin 3 (IL3).

4. Verfahren nach einem der Ansprüche 1 bis 3, worin die Polynucleotidsequenz ein Produkt kodiert, ausgewählt aus der Gruppe, bestehend aus einem Peptid, einem Ribozym und einer antisense Sequenz.

5. Verfahren nach einem der Ansprüche 1 bis 4, worin das Gen-Übermittlungs-Vehikel ausgewählt ist aus der Gruppe, bestehend aus einem retroviralen Vektor, einem DNS-Vektor und einem liposomalen Übermittlungs-Vehikel.

6. Verfahren zum Modifizieren einer hematopoietischen Stammzelle, umfassend das in Kontakt bringen eines Gen-Übermittlungs-Vehikels, umfassend eine Polynucleotidsequenz, mit einer Population hematopoietischer Stammzellen, die mit Fibronection oder RetroNectin™ und in Anwesenheit einer Menge an Thrombopoietin (TPO), einem flt3-Liganden (FL) und Interleukin 6 (IL6) kultiviert werden, die ausreicht, das Überleben, die Expansion und/oder die Transduktion der der hemtopoietischen Stammzellen zu fördern, worin: (i) das Verfahren kein Verfahren zur Behandlung des menschlichen oder tierischen Körpers mittels Chirurgie oder keine Therapie ist und (ii) das Verfahren humane Embryos nicht für industrielle oder kommerzielle Zwecke verwendet.

7. Verfahren nach Anspruch 6, zusätzlich umfassend das Kultivieren der Stammzellen in Anwesenheit des Leukämie inhibitorischen Faktors (LIF).

8. Verfahren nach Anspruch 6, zusätzlich umfassend das Kultivieren der Stammzellen in Anwesenheit von Interleukin 3 (IL3).

9. Verfahren nach Anspruch 6, zusätzlich umfassend das Kultivieren der Stammzellen in Anwesenheit eines c-kit Liganden.

10. Verfahren nach Anspruch 8, zusätzlich umfassend das Kultivieren der Stammzellen in Anwesenheit eines c-kit Liganden.

11. Verfahren nach Anspruch 6, worin die Polynukleotidsequenz ein Produkt kodiert, ausgewählt aus der Gruppe, bestehend aus einem Peptid, einem Ribozym und einer antisense Sequenz.

12. Verfahren nach Anspruch 6, worin das Gen-Übermittlungs-Vehikel ausgewählt ist aus der Gruppe bestehend aus einem retroviralen Vektor, einem DNS-Vektor und einem liposomalen Übermittlungs-Vehikel.

13. Verfahren nach einem der Ansprüche 1 bis 12, zusätzlich umfassend die Expansion der hematopoietischen Stammzellen in Kultur.

14. Verfahren nach Anspruch 13, zusätzlich umfassend das Kultivieren der Zellen mit Interleukin 3 (IL3).

15. Verfahren nach Anspruch 13, zusätzlich umfassend das Kultivieren der Zellen mit dem Leukämie inhibitorischen Faktor (LIF).

16. Verfahren nach Anspruch 13, zusätzlich umfassend das Kultivieren der Zellen mit einem c-kit Liganden.

17. Verfahren nach Anspruch 13, worin die hematopoietischen Stammzellen durch die Fähigkeit der Selbsterneuerung und die Fähigkeit, alle hematopoietischen Zelllinien entstehen zu lassen, **gekennzeichnet** sind.

18. Verfahren nach Anspruch 13, worin die hematopoietischen Stammzellen humane hematopoietische Stammzellen sind.

19. Verfahren nach Anspruch 18, worin die humanen hematopoietischen Stammzellen CD34⁺ sind.

20. Verfahren nach Anspruch 18, worin die humanen hematopoietischen Stammzellen CD34⁺Thy-1⁺ sind.

21. Verfahren nach Anspruch 18, worin die humanen hematopoietischen Stammzellen CD34⁺Thy-1⁺Lin⁻ sind.

## Revendications

1. Procédé pour modifier une cellule souche hématopoïétique, comprenant la mise en contact d'un véhicule de délivrance de gène comprenant une séquence nucléotide avec une population de cellules souches hématopoïétiques mises en culture avec de la fibronectine ou RetroNectin (marque commerciale) et en présence d'une quantité d'un mpl-ligand et d'un fit3-ligand (FL) qui est suffisant pour promouvoir la survie, l'expansion et/ou la transduction des cellules souches hématopoïétiques; dans lequel (i) le procédé n'est pas un procédé pour le traitement du corps humain ou animal par chirurgie ou thérapie ; et (ïi) le procédé n'utilise pas des embryons humains à des fins industrielles ou commerciales.

2. Procédé selon la revendication 1, comprenant de plus la mise en culture de cellules souches hématopoïétiques en présence d'un c-kit-ligand.

3. Procédé selon la revendication 2, comprenant de plus la mise en culture de cellules souches hématopoïétiques en présence d'interleukina 3 (IL3).

4. Procédé selon l'une quelconque des revendications 1 à 3, dans lequel la séquence polynucléotide code un produit choisi dans le groupe consistant en un peptide, un ribozyme et une séquence anti-sens.

5. Procède selon l'une quelconque des revendications 1 à 4, dans lequel le véhicule de délivrance de gènes est choisi dans le groupe consistant en un vecteur rétroviral, un vecteur ADN et un véhicule de délivrance liposomal.

6. Procédé pour modifier une cellule souche hématopoïétique, comprenant la mise en contact d'un véhicule de délivrance de gènes comprenant une séquence polynucléotide avec une population de cellules souches hématopoïétiques mises en culture avec de la fibronectine ou RetroNectin (marque commerciale) et en présence d'une quantité de thromhopoiétine (TPO), un flt3-ligand (FL), et l'interleukine 6 (IL6), qui est suffisant pour promouvoir la survie, l'expansion et/ou la transduction des cellules souches hématopoïétiques, dans lequel (i) le procédé n'est pas un procédé pour le traitement du corps humain ou animal par chirurgie ou thérapie ; et (ii) le procédé n'utilise pas les embryons humains à des fins industrielles ou commerciales.

7. Procédé selon la revendication 6, comprenant de plus la mise en culture des cellules souches en présence du facteur d'inhibition de la leucémie (LIF).

8. Procédé selon la revendication 6, comprenant de plus la mise en culture des cellules souches en présence d'interleukine 3 (IL3).

9. Procédé selon la revendication 6, comprenant de plus la mise en culture des Cellules souches en présence d'un c-kit-ligand.

10. Procédé selon la revendication 8, comprenant de plus la mise en culture des cellules souches en présence d'un c-kit-ligand.

11. Procédé selon la revendication 6, dans lequel la séquence polynucléotide code un produit choisi dans le groupe consistant en un peptide, un ribozyme et une séquence anti-sens.

12. Procédé selon la revendication 6, dans lequel le véhicule de délivrance de gène est choisi dans le groupe consistant en un vecteur rétroviral, un vecteur ADN et un véhicule de délivrance liposomal.

13. Procédé selon l'une quelconque des revendications 1 à 12, comprenant de plus l'expansion de cellules souches hématopoïétiques en culture.

14. Procédé selon la revendication 13, comprenant de plus la mise en culture des cellules avec l'interleukine 3 (IL3).

15. Procédé selon la revendication 13, comprenant de plus la mise en culture des cellules avec la facteur inhibiteur de la leucémie (LIF).

16. Procédé selon la revendication 13, comprenant de plus la mise en culture des cellules avec un c-kit-ligand.

17. Procédé selon la revendication 13, dans lequel les cellules souches hématopoïétiques sont **caractérisées par** la capacité d'auto - renouvellement et l'aptitude à donner naissance à toutes les lignées de cellules hématopoïétiques.

18. Procédé selon la revendication 13, dans lequel les cellules souches hématopoïétiques sont des cellules hématopoïétiques humaines.

19. Procédé selon la revendication 18, dans lequel les cellules souches hématopoïétiques humaines sont CD34⁺.

20. Procédé selon la revendication 18, dans lequel les cellules souches hématopoïétiques humaines sont CD34⁺Thy-1⁺.

21. Procédé selon la revendication 18, dans lequel les cellules souches hématopoïétiques sont CD34⁺Thy-1⁺Lin⁻.
